# EUROPEAN PATENT SPECIFICATION

(11) **EP 4 376 905 B1**
(45) Date of publication and mention of the grant of the patent: **22.10.2025**
(21) Application number: 22743514.6
(22) Date of filing: 25.07.2022
(51) Int. Cl.: A61L 24/04

(54) **COMPOSITIONS FOR EMBOLIZATION**
EMBOLISIERUNGSZUSAMMENSETZUNGEN
COMPOSITIONS POUR EMBOLISATION

(30) Priority: 26.07.2021 EP 21382685
(43) Date of publication of application: 05.06.2024
(73) Proprietor: Iberhospitex, S.A., 08185 Lliçà de Vall (ES)
(72) Inventor: LÓPEZ MOYA, Mario, 08420 CANOVELLES (ES); RAMOS PÉREZ, Víctor, 08185 LLIÇÀ DE VALL (ES)
(74) Representative: ZBM Patents - Zea, Barlocci & Markvardsen
(86) International application number: PCT/EP2022/070825
(87) International publication number: WO 2023/006679

(56) References cited:
- WO-A1-2020/098673
- WO-A1-2020/202210
- MOTTU F ET AL: "Iodine-containing cellulose mixed esters as radiopaque polymers for direct embolization of cerebral aneurysms and arteriovenous malformations", BIOMATERIALS, ELSEVIER, AMSTERDAM, NL, vol. 23, no. 1, 1 January 2002 (2002-01-01), pages 121 - 131, XP004322627, ISSN: 0142-9612, DOI: 10.1016/S0142-9612(01)00087-4

## Description

This application claims the benefit of European Patent Application no. EP21382685.2 filed July 26, 2021.

### Technical Field

The present invention relates generally to compositions for use in vascular conditions, more particularly compositions for embolization of blood vessels. The compositions comprise a polymer that transitions from a liquid to a solid upon being subject to physiological conditions and a radiopaque compound.

### Background Art

In many clinical situations, it is required to embolize blood vessels to treat vascular malformations, such as aneurysms, arteriovenous malformations or fistulas, to control bleeding, such as organ bleeding or gastrointestinal bleeding, or to ablate diseased tissue, such as tumors. Embolization of blood vessels can be performed with a variety of different materials, such as particulates, metallic coils and polymer compositions. Among these materials, polymer compositions present an excellent versatility as they can be introduced through a delivery device in the liquid state and transition to a solid state once in contact with the tissue or physiological fluid. Polymeric materials employed in polymer compositions include those that polymerize in situ at the delivery site, i.e. cyanoacrylate, and those that are dissolved in a biocompatible solvent and transition to a solid state (precipitate) at the delivery site, i.e. ethylene vinyl alcohol copolymer, cellulose acetate or polyacrylates.

Ideally, polymeric compositions for embolization should be easy to deliver through a catheter and should cause rapid and controllable embolization in the intended vascular site producing a space-filling and coherent mass. The resulting solid material must be sufficiently cohesive to avoid fragmentation, which may result in smaller solid materials incorporated in the circulation system and ultimately lead to embolization of undesired locations. In addition, polymeric compositions should be biocompatible and radiopaque. Radiopacity of embolizing compositions is important in order to monitor the injection of the composition in the vascular site and to confirm the completeness and the quality of the embolization procedure. Therefore, polymeric formulations include contrast agents as visualization species that can permit visualization in vivo, normally under fluoroscopic guidance.

US5667767 discloses an injectable liquid embolizing composition comprising an ethylene vinyl alcohol copolymer dissolved in dimethylsulfoxide or other suitable biocompatible solvent and a water insoluble radiopaque compound selected from tantalum, tantalum oxide, or barium sulfate. However, these compositions are difficult to handle. They require prolonged pre-mixing to obtain homogeneous suspension of the tantalum powder. Despite vigorous mixing, sedimentation of tantalum powder may compromise visual control during long injections. Moreover, tantalum may produce artifacts and causes excessive shielding during fluoroscopic monitoring, which is disadvantageous, particularly in the case of requiring repeated embolization reinterventions (which happens very frequently in the clinical practice). As for barium oxide, this compound has been additionally described to produce toxic effects.

Embolizing polymeric compositions containing water soluble contrast agents have also been suggested. Said water-soluble contrast agents are often iodine-based radiopaque compounds, such as lohexol (CAS no. 66108-95-0), lopamidol (CAS no. 60166-93-0) and lopromide (CAS no. 73334-07-3). However, the use of water-soluble contrast agents may limit the ability to monitor the injection procedure of the embolizing agent into the blood vessel due to rapid dissolution and removal from the site of injection. Highly water soluble contrast agents may also alter the precipitation behavior of the embolization formulation and/or the cohesiveness of the resulting plug, causing unintended embolization of distal blood vessels. In addition, the leaching of water-soluble contrast agent from the embolized region may arise the chance of local and systemic side effects in the treated subjects. For these reasons, highly water soluble contrast agents have been scarcely used for the embolization of blood vessels.

In order to solve the limitations mentioned above, covalently polymer-bonded iodine-based contrast agents have been developed to ensure homogeneous radiopacity over time without the need of premixing. For example, EP2906254 discloses injectable embolizing compositions which comprise a biocompatible polymer including a biodegradable linkage to a radiopaque iodinated compound, and a non-physiological solution. The liquid composition precipitates when contacting physiological conditions within the blood vessel. However, these compositions require complex synthesis. And, besides, covalent conjugation of iodine-based contrast agents may have a deleterious effect in the precipitation behavior and/or cohesiveness of the embolization composition.

WO 2020/098673 A1 discloses a liquid polymeric composition comprising a polymer such as ethylene vinyl alcohol copolymer, a radiopaque molecule such as 2,4,6-triiodophenol, 2,3,5-triiodobenzoic acid, 2,3,5-sodium triiodobenzoate, iodotitanic acid, formazan acid, iododonic acid or iohexol with a molecular weight below 2000 g/mol, and a non-physiological solvent system or solvent system such as dimethyl sulfoxide or N-methylpyrrolidone, wherein the polymer and the radiopaque molecule are both dissolved in the non-physiological solvent or solvent system. The composition may be used for embolization by injecting it into a physiological environment whereby it precipitates and embolizes.

There is a need to provide liquid embolizing compositions that do not have the disadvantages mentioned above.

### Summary of Invention

The inventors have found that injectable embolizing compositions having the desired properties can be conveniently prepared by dissolving a polymer and selected radiopaque molecules in a non-physiological solution.

Thus, the first aspect of the invention refers to a liquid polymeric composition comprising: (a) from about 2 to about 20 weight percent of a polymer selected from the group consisting of ethylene vinyl alcohol copolymer, cellulose acetate, poly(2-hydroxyethylmethacrylate), copolymers of cellulose acetate, poly(2-hydroxyethylmethacrylate), or both, poly(ethylene oxide)-co-poly(propylene oxide) copolymers, poly(oligoethylene glycol)-(meth)acrylates, poly(meth)acrylate copolymers, poly(meth)acrylamide copolymers, and combinations thereof; (b) from about 5 to about 40 weight percent of a radiopaque molecule with a molecular weight below 2000 g/mol; (c) from about 40 to about 93 weight percent of a non-physiological solvent or solvent system; wherein: (i) the sum of the weight percent of all components in the composition is 100, (ii) the polymer and the radiopaque molecule are both dissolved in the non-physiological solvent or solvent system and (iii) the liquid polymeric composition is capable of forming, upon contact with physiological conditions, a radiopaque precipitate.

Precipitation of the polymer occurs upon injection where the solvent diffuses into the surrounding aqueous environment and water diffuses into the polymer matrix, which leads to a solid polymeric implant in situ (or embolus), embedding the radiopaque compound, thus rendering a cohesive, homogeneous radiopaque precipitate. The formed radiopaque precipitate entraps a sufficient amount of radiopaque material to allow appropriate radioscopic monitoring/identification of the procedure. The radiopaque compound is then gradually released from the precipitate into the physiological fluid (e.g. into the blood circulation) and consequently the precipitate loses radiopacity in a gradual manner. Eventually, the radiopacity vanishes completely and the embolized area becomes translucent to fluoroscopy. For example, the polymeric composition of the invention forms a radiopaque precipitate that maintains its radiopacity for a convenient range of time, for example, the radiopaque precipitate may maintain at least 50% of its radiopacity during at least 30 minutes and lose at least 50% of its radiopacity in less than 6 months.

The liquid polymeric compositions of the first aspect of the invention are herein also called "embolizing compositions" or, simply, "compositions" of the invention.

The polymer is soluble in the non-physiological solvent or solvent system and insoluble in a physiological solution. Upon contact with physiological conditions, for example, when injected into a blood vessel, the polymer in the composition precipitates embedding the radiopaque compound.

The radiopaque compound of the composition of the invention is not covalently bound to the polymer, such that the embolizing compositions are easy and economical to prepare. Further, said radiopaque molecule, as well as the polymer, is dissolved in the liquid embolizing composition. Consequently, these compositions are easy to handle and do not require mixing to homogenize the radiopaque molecule in the composition. There is also no risk of the radiopaque compound sedimenting before or during injection to the patient.

The liquid polymeric compositions of the invention are easily delivered to the vascular site and rapidly forms a cohesive and homogeneous solid precipitate whose radiopacity decreases with time. Importantly, the liquid polymeric compositions of the invention allow for adequate monitoring of the formed embolus during the embolizing procedure and may bioresorb in a clinically relevant time frame to allow better monitoring in those cases requiring reintervention.

The compositions according to the first aspect of the invention are prepared by conventional methods whereby each of the components is added and the resulting composition mixed together until the overall composition is substantially homogeneous.

Thus, in a second aspect, the invention also refers to a method for preparing a liquid polymeric composition as defined in the first aspect of the invention, the method comprising the steps of: (i) providing a non-physiological solvent or solvent system, a polymer and a radiopaque compound and (ii) mixing until a homogeneous dissolution is achieved.

A third aspect of the invention refers to an injectable (product) comprising a liquid polymeric composition as defined in the first aspect of the invention.

A fourth aspect of the invention refers to a kit for embolization, said kit comprising a container containing a liquid polymeric composition according to the first aspect of the invention.

A fifth aspect of the invention refers to a liquid polymeric composition as defined in the first aspect for use in embolizing a blood vessel. This may also be formulated as a method for embolizing a blood vessel by injecting into said blood vessel a sufficient amount of a liquid polymeric composition as defined in the first aspect.

A sixth aspect of the invention refers to a liquid polymeric composition as defined in the first aspect for use in filling a vascular defect. This may also be formulated as a method for filling a vascular defect by injecting a sufficient amount of a liquid polymeric composition as defined in the first aspect.

### Brief Description of Drawings

Fig. 1 shows exemplary radiopaque molecules
Fig. 2 shows radiopacity of different formulations after 30 min and 30 days of incubation in PBS at 37°C.
Fig 3 shows *in vivo* radiopacity loss of compositions containing radiopaque compounds implanted subdermally in a New Zealand rabbit.
Fig. 4. Embolized kidneys with formulation LFE09 at time zero (right) and after 14 days (left).
Fig 5. Radiopacity loss of radiopaque precipitates vs logP of radiopaque molecules in vivo. Mice (n = 3 for each group) were injected with 30 ul of embolization formulations containing a) Tantalum at 300 mg/ml, b) LFE01 (logP 6.8) at 150 mg equiv. of iodine per ml c) LFE04 (logP 3.9) at 150 mg equiv. of iodine per ml or d) iomeprol (logP -2.8) at 150 mg equiv. of iodine per ml. Mice were euthanized at day 1 after injection, day 15 and day 30 and the radiopacity of their radiopaque implants was assessed by fluoroscopy imaging.

Radiopacity loss was determined following the standard method ASTM F640-20 Standard Test Methods for Determining Radiopacity for Medical Use.

### Detailed description of the invention

Prior to discussing this invention in further detail, the following terms will first be defined:
As used herein, the indefinite articles "a" and "an" are synonymous with "at least one" or "one or more." Unless indicated otherwise, definite articles used herein, such as "the" also include the plural of the noun.

The term "embolizing" refers to a process wherein a material is injected into a blood vessel which thereafter fills or plugs the blood vessel and/or encourages clot formation so that blood flow through the vessel ceases. The embolization of the blood vessel is important in preventing/controlling bleeding (e.g., organ bleeding, gastrointestinal bleeding, vascular bleeding, bleeding associated with an aneurysm) or to ablate diseased tissue (e.g., tumors, etc.) by cutting off its blood supply.

The term "radiopaque molecule", herein sometimes also referred to as "contrast agent", refers to a molecule that will not allow X-rays or similar radiation to pass. Radiopacity may be determined by standard methods in vivo or in vitro. In vivo it is common to determine radiopacity by radiography.

The term "biocompatible" refers to an organic material which performs with an appropriate host response in a specific application. Biocompatible materials preferably do not produce adverse effects when contacting the body in the amounts used.

The expression "physiological conditions" refers to conditions within or compatible with the human (or generally, mammal) body, particularly within the vascular system. A skilled person in the art may readily recognize which conditions are considered to be physiological. For example, physiological conditions include a temperature range of 35-40 degrees Celsius, atmospheric pressure of 1, pH of 6-8, in particular around 7.5, and glucose concentration of 1-20 mM. More particularly, *in vitro* physiological conditions are understood herein as phosphate buffered saline at pH 7.4 and 37°C. Non-physiological conditions are consequently those conditions which are not compatible with the human body. Again, the skilled person can readily recognize conditions that are not physiological.

The term "ethylene vinyl alcohol copolymers" refers to copolymers comprising residues of both ethylene and vinyl alcohol monomers. Small amounts (e.g., less than 5 mole percent) of additional monomers can be included in the polymer structure or grafted thereon provided such additional monomers do not alter the embolizing properties of the composition. Such additional monomers include, by way of example only, maleic anhydride, styrene, propylene, acrylic acid, vinyl acetate and the like.

The expression "weight percent" or "% by weight" or "% wt" is understood as the weight percentage of the referred ingredient with respect to the total amount of the final composition. In the present invention weight percent usually refers to % w/v, in other words, x % by weight of ingredient A represents x grams of ingredient A in 100 ml of the total composition. The sum of the % wt of the ingredients in the composition must be 100.

The term "functional group" is understood as generally in the state of the art, i.e., as a specific group of atoms or bonds within a molecule that is responsible for the characteristic chemical reactions of that molecule. The same functional group will undergo the same or similar chemical reaction(s) regardless of the size of the molecule it is a part of.

The expression "(Cₓ-C_{y})-alkyl" as used herein refers to a saturated branched or linear hydrocarbon side chain with x to y carbon atoms. For example, "(Cₓ-C_{y})-alkyl" may be (C₁-C₄)-alkyl, which is preferably an unsubstituted group selected from methyl, ethyl, propyl, isopropyl, butyl, isobutyl, s-butyl, and t-butyl.

### Compositions

Described herein are liquid polymeric compositions comprising a polymer and a radiopaque molecule dissolved in a non-physiological solvent or solvent system. These compositions can be introduced into a physiological fluid through a delivery device in the liquid state and transition to the solid state once in contact with the physiological fluid. The compositions are useful for embolizing blood vessels.

The compositions of the first aspect of the invention comprise: (a) from about 2 to about 20 weight percent of a polymer selected from the group consisting of ethylene vinyl alcohol copolymer, cellulose acetate, poly(2-hydroxyethylmethacrylate), copolymers of cellulose acetate, poly(2-hydroxyethylmethacrylate), or both, poly(ethylene oxide)-co-poly(propylene oxide) copolymers, poly(oligoethylene glycol)-(meth)acrylates, poly(meth)acrylate copolymers, poly(meth)acrylamide copolymers, and combinations thereof; (b) from about 5 to about 50 weight percent of a radiopaque molecule; (c) from about 40 to about 93 weight percent of a non-physiological solvent or solvent system.

In one embodiment of the first aspect of the invention, optionally in combination with any of the embodiments provided above or below, the composition of the invention comprises: (a) from about 2 to about 20 weight percent of a polymer; (b) from about 5 to about 40 weight percent of a radiopaque molecule; (c) from about 40 to about 93 weight percent of a non-physiological solvent or solvent system.

In one embodiment of the first aspect of the invention, optionally in combination with any of the embodiments provided above or below, the composition of the invention comprises: (a) from about 2.5 to about 8 weight percent of a polymer; (b) from about 10 to about 40 weight percent of a radiopaque molecule; (c) from about 52 to about 87.5 weight percent of a non-physiological solvent or solvent system.

In another embodiment, the composition of the invention comprises: (a) from about 4 to about 5.2 weight percent of a polymer; (b) from about 20 to about 40 weight percent of a radiopaque molecule; (c) from about 54.8 to about 76 weight percent of a non-physiological solvent or solvent system.

The sum of the weight percent of all components in the composition is 100.

As mentioned above, both the polymer and the radiopaque compound are independently dissolved in the non-physiological solvent. The radiopaque molecule has no covalent link to the polymer. Upon contact with physiological conditions, for example, when injected into a blood vessel, the polymer in the composition precipitates entrapping the radiopaque compound, thus forming a radiopaque precipitate. Preferably, the radiopaque molecule is homogeneously distributed within the precipitate. The contrast agent and polymer form an integral coherent precipitate which does not separate into a copolymer component and a contrast agent component. A sufficient amount of the radiopaque molecule remains within the precipitate upon solidification for a sufficient amount of time to allow appropriate monitoring of the embolization procedure. Subsequently, the radiopaque molecule is gradually released from the embolized region/formed precipitate and as a result the radiopacity vanishes gradually.

In some embodiments of the first aspect of the invention, optionally in combination with any of the embodiments provided above or below, the precipitate maintains at least 50% of its radiopacity during at least 24 hours, or maintains at least 50% of its radiopacity during at least 8 hours, or maintains at least 50% of its radiopacity during at least 4 hours, or maintains at least 50% of its radiopacity during at least 2 hours, or maintains at least 50% of its radiopacity during at least 1 hour, or maintains at least 50% of its radiopacity during at least 30 minutes.

In some embodiments of the first aspect of the invention, optionally in combination with any of the embodiments provided above or below, the precipitate loses at least 50% of its radiopacity in less than 6 months, or loses at least 50% of its radiopacity in less than 5 months, or loses at least 50% of its radiopacity in less than 4 months, or loses at least 50% of its radiopacity in less than 3 months, or loses at least 50% of its radiopacity in less than 3 months, or loses at least 50% of its radiopacity in less than 1 month, or loses at least 50% of its radiopacity in less than 15 days.

In particular embodiments of the first aspect of the invention, optionally in combination with any of the embodiments provided above or below, the radiopaque precipitate maintains at least 50% of its radiopacity during at least 30 minutes and loses at least 50% of its radiopacity in less than 6 months. In particular embodiments, the radiopaque precipitate maintains at least 50% of its radiopacity during at least 30 minutes and loses at least 50% of its radiopacity in less than 3 months. In other particular embodiments, the radiopaque precipitate maintains at least 50% of its radiopacity during at least 30 minutes and loses at least 50% of its radiopacity in less than 1 months. In other particular embodiments, the radiopaque precipitate maintains at least 50% of its radiopacity during at least 1 hour and loses at least 50% of its radiopacity in less than 3 months. In other particular embodiments, the radiopaque precipitate maintains at least 50% of its radiopacity during at least 1 hour and loses at least 50% of its radiopacity in less than 1 month. In other particular embodiments, the radiopaque precipitate maintains at least 50% of its radiopacity during at least 24 hours and loses at least 50% of its radiopacity in less than 15 days.

In some embodiments of the first aspect of the invention, optionally in combination with any of the embodiments provided above or below, the radiopaque molecule is continuously released from the formed precipitate for up to 6 months. In particular embodiments, the radiopaque molecule is continuously released from the formed precipitate for up to 3 months. In more particular embodiments, the radiopaque molecule is continuously released from the formed precipitate for up to 1 month.

The skilled person may determine radiopacity loss by standard methods in vivo or in vitro. For example, radiopacity may be measured by injecting a liquid polymeric embolizing composition in saline and determining radiopacity of the formed precipitate with fluoroscopy along time. Example 2 below shows an exemplary protocol for evaluating radiopacity loss of an embolizing composition and the results observed for several liquid polymeric compositions according to the invention. Radiopacity loss can be determined by image analysis of X-Ray images of the embolus at different time points, as described in the standard method ASTM F640-20 Standard Test Methods for Determining Radiopacity for Medical Use. The embolus formed by the precipitate is usually visualized in vivo under fluoroscopy (X-Ray) and/or computed tomography (CT) imaging.

The liquid polymeric compositions of the invention are deployed through a delivery device, e.g. microcatheter, to a delivery site and/or treatment site. The viscosity of the compositions must allow for delivery through such device. In some embodiments of the first aspect of the invention, optionally in combination with any of the embodiments provided above or below, the liquid polymeric composition has a viscosity equal to or less than 40 centistoke at 40°C. In particular, the viscosity of the liquid polymeric composition is 30 centistoke or less at 40°C. In some embodiments of the first aspect of the invention, optionally in combination with any of the embodiments provided above or below, the liquid polymeric composition has a viscosity equal or above 10 centistoke at 40°C.

### Radiopaque molecules

The inventors have found that an optimal monitoring of the embolization procedure is achieved when the radiopaque compound in the composition has a LogP from 2.0 to 9.0, in particular when the radiopaque compound has a LogP from 2 to 7.

The term LogP refers to the partition coefficient of the contrast agent molecule between an aqueous and octanol phases. A skilled person knows how to determine the LogP following standard empirical procedures (Chemical Reviews, 1971, Vol. 71, No. 6, 525 - 616). However, nowadays, it is more usual to determine the LogP using software. Appropriate software to determine LogP are commercially available. For example, the LogP of the radiopaque compound of the invention may be determined with ChemAxon, version 20.7.0.

Radiopaque molecules having LogP values between 2 and 9, in particular from 2 to 7, remain entrapped in the precipitate during the embolization procedure, allowing excellent monitoring under fluoroscopy, without the need of being covalently bound to the polymer. However, such molecules are slowly released from the precipitate, allowing gradual radiopacity loss and avoiding the problems associated with common contrast agents such as tantalum or ethiodized oil.

Moreover, the radiopaque molecules of the composition of the invention have no deleterious effect in the precipitate, such that the compositions containing said radiopaque molecules form a cohesive precipitate with no fragmentation.

In one embodiment of the first aspect of the invention, optionally in combination with any of the embodiments provided above or below, the radiopaque molecule has a LogP from 2.0 to 7.0. In a particular embodiment of the first aspect of the invention, optionally in combination with any of the embodiments provided above or below, the radiopaque molecule has a LogP from 2.0 to 6.0. In a particular embodiment of the first aspect of the invention, optionally in combination with any of the embodiments provided above or below, the radiopaque molecule has a LogP from 3.0 to 5.5, or from 3.0 to 5.0, or from 3.5 to 5, or from 4 to 5.

According to the present invention, the LogP of the radiopaque compound is preferably considered in the form acquired by said compound in the composition. In this sense, it is noted that some radiopaque molecules, for example, those that are susceptible of ionization, may suffer transformations when subjected to physiological conditions that result in a change of LogP. For example, a radiopaque compound may become protonated when subjected to physiological conditions, thus modifying its LogP. Other radiopaque molecules may contain biodegradable linkages, such as esters, polyesters, carbonates or peptides, which are capable of being chemically or enzymatically hydrolyzed in a physiological environment. Hydrolysis of said linkages may result in lowering of the LogP to fall within the required range in the precipitate.

In one particular embodiment of the first aspect of the invention, optionally in combination with any of the embodiments provided above or below, the radiopaque molecule is a non-ionizable molecule. In particular embodiments, the non-ionizable radiopaque compound has in the composition a LogP from 2.0 to 7.0, in particular from 3.0 to 5.0.

In another particular embodiment of the first aspect of the invention, optionally in combination with any of the embodiments provided above or below, the radiopaque molecule is an ionizable molecule. In particular embodiments, the ionizable radiopaque compound has in the composition a LogP from 4.0 to 9.0, in particular from 5.0 to 8.0. In particular embodiments, said ionizable radiopaque molecule comprises acidic or basic groups. When the radiopaque compound is susceptible of being ionized, for example, because it contains acidic or basic groups, the LogP of the radiopaque compound may be considered in the form acquired by said compound in physiological conditions, that is, conditions resembling what happens when the compound is injected into a blood vessel. In this sense, the ionizable radiopaque compound may have a LogP that is in the range from 2.0 to 7.0, in particular from 3.0 to 5.0, when subjected to physiological conditions.

In a particular embodiment of the first aspect of the invention, optionally in combination with any of the embodiments provided above or below, the radiopaque molecule is liquid at room pressure and room temperature.

In most embodiments of the first aspect of the invention, the radiopaque molecule is an aromatic compound. In most embodiments of the first aspect of the invention, the radiopaque molecule is an iodinated compound. In some embodiments of the first aspect of the invention, optionally in combination with any of the embodiments provided above or below, the radiopaque molecule is an aromatic iodinated compound, for example, an aromatic organic molecule containing iodine or bromine at concentration ranging from 200 to 900 mg equivalent of iodine or bromine per gram of molecule. In particular embodiments the radiopaque molecule has a concentration ranging from 300 to 800 mg equivalent of iodine or bromine per gram of molecule, or ranging from 350 to 700 mg equivalent of iodine or bromine per gram of molecule, or ranging from 400 to 600 mg equivalent of iodine per gram of molecule. The radiopaque molecule in the compositions of the invention provides for an iodine or bromine concentration in the composition that can range from about 50 to about 500 mg equivalents of iodine or bromine/ml composition, or from about 100 to about 300 mg equivalents of iodine or bromine/ml, or from about 100 to about 250 mg equivalents of iodine or bromine/ml, or about 125 to about 200 mg equivalents of iodine or bromine/ml, for example 150 mg equivalents of iodine or bromine/ml. In particular embodiments of the first aspect of the invention, optionally in combination with any of the embodiments provided above or below, the radiopaque molecule is an aromatic organic molecule with at least two iodine or bromine atoms. In particular embodiments of the first aspect of the invention, optionally in combination with any of the embodiments provided above or below, the radiopaque molecule contains iodine. Aromatic rings with a plurality of iodine atoms provide a good option in contrast media design. Aromatic rings appropriate in the sense of the present invention may have five or six Carbons. Non-limiting examples of aromatic rings are benzene, pyridone, and pyridine. For example, appropriate aromatic rings containing iodine can be triiodophenol, iophenoxic acid, triiodobenzoic acid, 3,5-diiodo-4-pyridone, triiodoaniline, and derivatives thereof, for example, esters and ethers of triiodophenol, iophenoxic acid, triiodobenzoic acid, 3,5-diiodo-4-pyridone, or triiodoaniline. Such compounds exhibit higher stability and lower toxicity than iodinated aliphatic molecules. Particularly convenient contrast agents produce a homogeneous and coherent precipitate together with the polymer.

The contrast agents' physical, chemical, and pharmacological properties can be manipulated and modified by exploiting the functional groups present on the aromatic ring. In some embodiments of the first aspect of the invention, optionally in combination with any of the embodiments provided above or below, the compositions conveniently contain contrast agents whose molecular structure present an amphiphilic character, with clearly defined hydrophilic and hydrophilic parts. In particular embodiments of the first aspect of the invention, optionally in combination with any of the embodiments provided above or below, the radiopaque compound is selected from the group consisting of a pegylated derivative of triiodophenol, a pegylated derivative of iophenoxic acid, and a pegylated derivative of triiodobenzoic acid.

As mentioned above, in some embodiments of the first aspect of the invention, the contrast agents include at least one linkage susceptible of being cleaved in physiological conditions. Such biodegradable linkages/bonds within the structure may be broken in physiological conditions, releasing part of the original structure as a molecule with different solubility. Thus, in some embodiments of the first aspect of the invention, optionally in combination with any of the embodiments provided above or below, the radiopaque molecule is transformed in physiological conditions into a molecule that has a LogP which is appropriate in the sense of the present invention as described above. Biodegradable linkages can be generally separated into two types, those susceptible to hydrolysis and those susceptible to enzymatic action. Linkages susceptible to hydrolysis can generally include esters, polyesters, or carbonates. One skilled in the art can envision feasible methods of introducing biodegradable linkages into the contrast agent molecule. Ester bonds can be introduced by reacting hydroxyl groups with anhydrides or acid chlorides, among others. The rate of radiopacity loss can be controlled, for example, by ester selection and the number of esters inserted into the contrast agent. Linkages capable of being enzymatically hydrolyzed in a physiological environment can include peptide bonds that can be degraded by enzymes, such as, but not limited to, matrix metalloproteinases, collagenases, elastases, cathepsin, or a combination thereof.

In particular embodiments of the first aspect of the invention, optionally in combination with any of the embodiments provided above or below, the radiopaque molecule is a compound of Formula I or Formula II 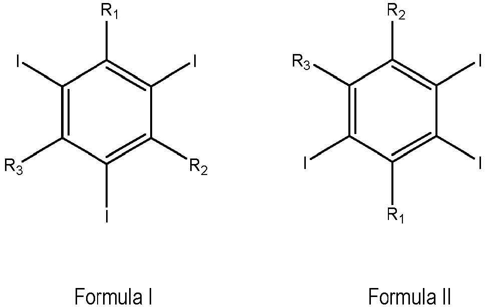 wherein:
R2 and R3 are independently selected from the group consisting of -H, -COOH, -(C₁-C₂₅)alkyl, -(C₁-C₂₅)alkyl-COOH, -O-(C₁-C₂₅)alkyl, -(C₁-C₂₅)alkyl-COO-(C₁-C₂₅)alkyl, -O-(CH₂-CH₂-O)_{b}-CH₃,-NH-CO-(C₁-C₂₅)alkyl, -NH₂, -NH-(C₁-C₂₅)alkyl, -N-((C₁-C₁₀)alkyl)₂ -CF₃, -Cl, -F, -CN, -NO₂, and -CO-NH-(C₁-C₂₅)alkyl,
R1 is independently selected from the group consisting of:

   -X-(C₁-C₂₀)alkyl-COO-R4

   -X-(CH₂-CH₂-O)_{b}-R4,

   -X-(CH₂-CH₂-O)_{b}-(CH₂)_{c}-COO-R4,

   -X-(CH₂-CH₂-O)_{b}-COO-R4,

   -X-(C₁-C₁₀)alkyl-COO-(CH₂-CH₂-O)_{b}-R4,

   -X-(C₁-C₂₀)alkyl-NH₂,

   -X-(C₁-C₁₀)alkyl-NH-(C₁-C₁₀)alkyl

   -X-(C₁-C₁₀)alkyl-N-((C₁-C₁₀)alkyl)₂

   -(CH₂)_{c}-O-(CH₂-CH₂-O)_{b}-R4,
wherein
X is independently selected from the group consisting of O, COO and C(O)NH
R4 is selected from the group consisting of -H, -OH, -(C₁-C₈)alkyl, -(C₁-C₈)alkyl-OH, (C₁-C₈)acyl, -(CH₂-CH₂-O)_{b}-CH₃, and an alkyl chain containing at least one linkage capable of being chemically or enzymatically hydrolyzed in a physiological environment,
b is an integer from 0 to 20, and
c is an integer from 0 to 10.

In particular embodiments, c is an integer from 1 to 4, for example, 1 or 2.

In particular embodiments b is an integer from 1 to 15, for example, from 3 to 12.

In particular embodiments, R2 and R3 are independently selected from the group consisting of H, -(C₁-C₄)alkyl, -NH-CO-CH₃, -COOH, -COOCH₃, -COOCH₂CH₃, -NH₂, -NH-(C₁-C₄)alkyl, -N-((C₁-C₄)alkyl)₂ -CF₃, -Cl, -F, -CN, -NO₂, and -CO-NH-(C₁-C₄)alkyl.

In particular embodiments, R4 is selected from the group consisting of -H, -OH, -(C₁-C₄)alkyl, -(C₁-C₄)alkyl-OH, (C₁-C₄)acyl, -(CH₂-CH₂-O)_{b}-CH_{3,} -(C₁-C₄)alkyl-COO-(C₁-C₈)alkyl, and -(C₁-C₄)alkyl-C(O)NH-(C₁-C₈)alkyl. In particular embodiments, R4 is selected from the group consisting of -H, -OH, -(C₁-C₂)alkyl, -(C₁-C₂)alkyl-OH, (C₁-C₂)acyl, -(CH₂-CH₂-O)_{b}-CH₃, -CH₂-COO-CH₃, -CH₂-COO-CH₂-CH₃, -CH₂-CH₂-COO-CH₃, -CH₂-CH₂-COO-CH₂-CH₃, -CH₂-CONH-CH₃, -CH₂-CONH-CH₂-CH₃, -CH₂-CH₂-CONH-CH₃, and -CH₂-CH₂-CONH-CH₂-CH₃.

In particular embodiments, the radiopaque molecule is a compound of formula I.

In particular embodiments of the first aspect of the invention, optionally in combination with any of the embodiments provided above or below, the contrast agent is selected from the group consisting of a compound of formula III and a compound of formula IV:

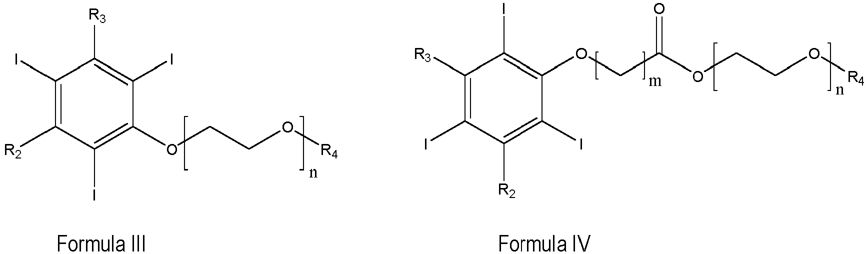

Wherein,
n and m are independently an integer from 1 to 20
R2 and R3 are independently selected from the group consisting of -H, -COOH, -(C₁-C₂₅)alkyl, -(C₁-C₂₅)alkyl-COOH, -O-(C₁-C₂₅)alkyl, -(C₁-C₂₅)alkyl-COO-(C₁-C₂₅)alkyl, -O-(CH₂-CH₂-O)_{b}-CH₃,-NH-CO-(C₁-C₂₅)alkyl, -NH₂, -NH-(C₁-C₂₅)alkyl, -N-((C₁-C₁₀)alkyl)₂ -CF₃, -Cl, -F, -CN, -NO₂, and -CO-NH-(C₁-C₂₅)alkyl,
R4 is selected from the group consisting of from -H, -OH, -(C₁-C₈)alkyl, -(C₁-C₈)alkyl-OH, (C₁-C₈)acyl, -(CH₂-CH₂-O)b-CH₃, wherein b is an integer from 0 to 20, and an alkyl chain containing at least one linkage capable of being chemically or enzymatically hydrolyzed in a physiological environment.

In some embodiments the radiopaque molecule contains a terminal hydroxyl.

In some embodiments, for any of the compounds of formula IV or III, n is an integer from 3 to 12. In some embodiments m is an integer from 1 to 5 . In some embodiments, R2 and R3 are independently selected from the group consisting of H, -(C₁-C₄)alkyl, -NH-CO-CH₃, -COOH, -COOCH₃, and -COOCH₂CH₃. In some embodiments, R4 is selected from the group consisting of -H, -OH, -(C₁-C₄)alkyl, -(C₁-C₄)alkyl-OH, (C₁-C₄)acyl, -(CH₂-CH₂-O)_{b}-CH₃, and an alkyl chain containing at least one linkage capable of being chemically or enzymatically hydrolyzed in a physiological environment, for example -(C₁-C₄)alkyl-COO-(C₁-C₈)alkyl and - (C₁-C₄)alkyl-C(O)NH-(C₁-C₈)alkyl. In some embodiments R4 is - H, -CH₃, -CH₂-COO-CH₃, -CH₂-COO-CH₂-CH₃, -CH₂-CH₂-COO-CH₃, -CH₂-CH₂-COO-CH₂-CH₃, -CH₂-CONH-CH₃, -CH₂-CONH-CH₂-CH₃, -CH₂-CH₂-CONH-CH₃, and -CH₂-CH₂-CONH-CH₂-CH₃. In some embodiments, the radiopaque molecule is a compound of formula III wherein R2 and R3 are both -H, n is 11, and R4 is -CH₃. In some embodiments, the radiopaque molecule is a compound of formula IV wherein R2 and R3 are both -H, n is 3, m is 1 and R4 is -CH₃.

In some embodiments of the first aspect of the invention, optionally in combination with any of the embodiments provided above or below, the contrast agents are sensitive to pH. Non-limiting examples of pH-sensitive radiopaque molecules are amino-containing iodinated compounds. Amino-containing iodinated compounds can be dissolved in deprotonated form in the compositions of the invention, such compounds may possess low water solubility and become entrapped in the formed precipitate during the embolization procedure. Once the precipitate is formed, the surrounding pH may cause the amines to substantially get protonated, causing a decrease in the LogP of the contrast agent that results in removal from the precipitate by diffusion into the surrounding physiological medium. In addition to some compounds that are included within the formulas provided above, other non-limiting examples of this type of radiopaque molecules are derivatives of 3,5-diiodo-4-pyridone-1-acetic acid and triiodoaniline. Thus, in other particular embodiments, the radiopaque molecule is a derivative of 3,5-diiodo-4-pyridone-1-acetic acid or triiodoaniline, for example, amides or N-acylated derivatives.

In some embodiments of the first aspect of the invention, optionally in combination with any of the embodiments provided above or below, the radiopaque molecule is a compound of formula V: 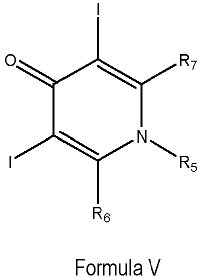 wherein,
R6 and R7 are independently selected from the group consisting of -H, -COOH, -(C₁-C₂₅)alkyl, -(C₁-C₂₅)alkyl-COOH, -O-(C₁-C₂₅)alkyl, -(C₁-C₂₅)alkyl-COO-(C₁-C₂₅)alkyl, -O-(CH₂-CH₂-O)_{b}-CH₃,-NH-CO-(C₁-C₂₅)alkyl, -NH₂, -NH-(C₁-C₂₅)alkyl, -N-((C₁-C₁₀)alkyl)₂ -CF₃, -Cl, -F, -CN, -NO₂, and -CO-NH-(C₁-C₂₅)alkyl, and
R5 is -(CH₂)_{d}-COO-R8, wherein d is an integer from 0 to 10 and R8 is selected from the group consisting of - H, -OH, -(C₁-C₈)alkyl, -(C₁-C₈)alkyl-OH, (C₁-C₈)acyl, -(CH₂-CH₂-O)_{b}-CH₃, wherein b is an integer from 0 to 20, and an alkyl chain containing at least one linkage capable of being chemically or enzymatically hydrolyzed in a physiological environment.

In some embodiments, d is an integer from 1 to 5, or from 1 to 4, for example 1, 2, 3 or 4. In some embodiments, R6 and R7 are independently selected from the group consisting of H, -(C₁-C₄)alkyl, -NH-CO-CH₃, -COOH, -COOCH₃, and -COOCH₂CH₃. In some embodiments R8 is selected from the group consisting of -H, -OH, -(C₁-C₄)alkyl, -(C₁-C₄)alkyl-OH, (C₁-C₄)acyl, -(CH₂-CH₂-O)_{b}-CH₃, -(C₁-C₄)alkyl-COO-(C₁-C₈)alkyl, and -(C₁-C₄)alkyl-C(O)NH-(C₁-C₈)alkyl. In some embodiments, the radiopaque molecule is a compound of formula V wherein R6 and R7 are both -H, d is 1, and R8 is -CH₂-CH₂-CH₃. In some embodiments, the radiopaque molecule is a compound of formula V wherein R6 and R7 are both -H, d is 1, and R8 is -(CH₂)₅-CH₃.

In certain embodiments of the first aspect of the invention, for any of the compounds of formula I to V, R4 and R8 may be an alkyl chain containing at least one linkage capable of being chemically or enzymatically hydrolyzed in a physiological environment. In particular embodiments, said linkage capable of being chemically hydrolyzed in a physiological environment is selected from esters, polyesters, amides, and carbonates, for example -(C₁-C₄)alkyl-COO-(C₁-C₈)alkyl or -(C₁-C₄)alkyl-C(O)NH-(C₁-C₈)alkyl. The linkage capable of being enzymatically hydrolyzed in a physiological environment can include peptide bonds that can be degraded by enzymes, such as, but not limited to, matrix metalloproteinases, collagenases, elastases, cathepsin, or a combination thereof.

In particular embodiments of the first aspect of the invention, optionally in combination with any of the embodiments provided above or below, the radiopaque molecule is a molecule selected form the compounds of formula I to V that contains at least one pegylated chain.

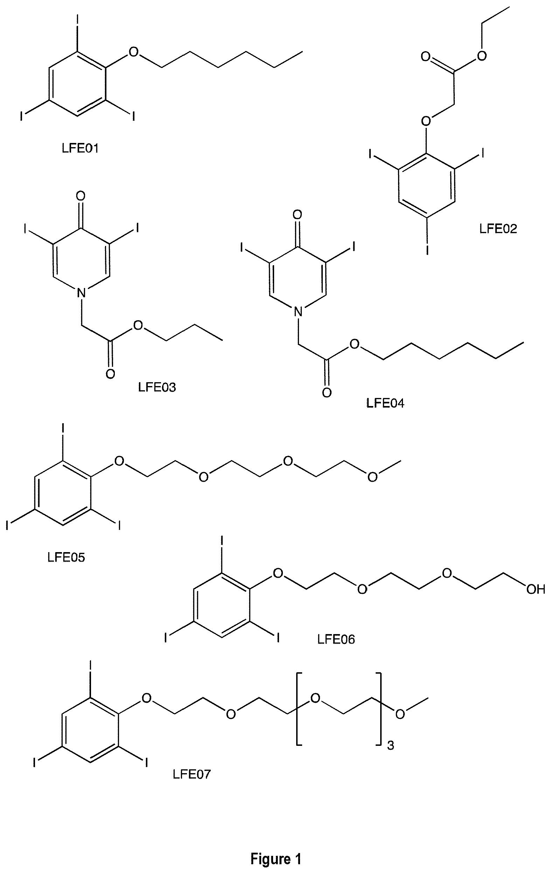

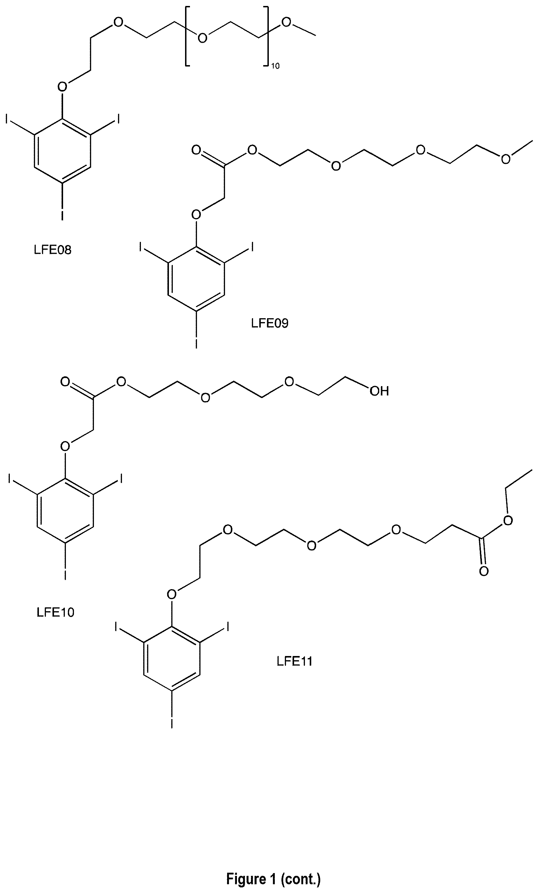

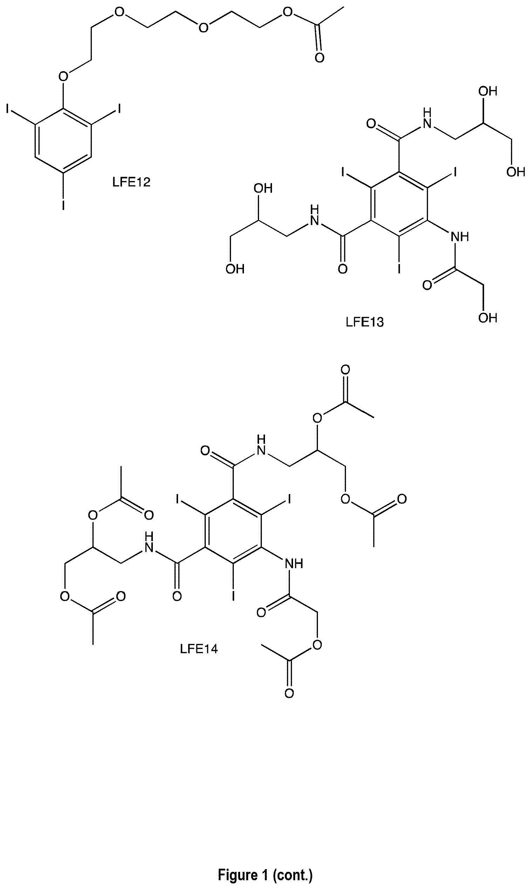

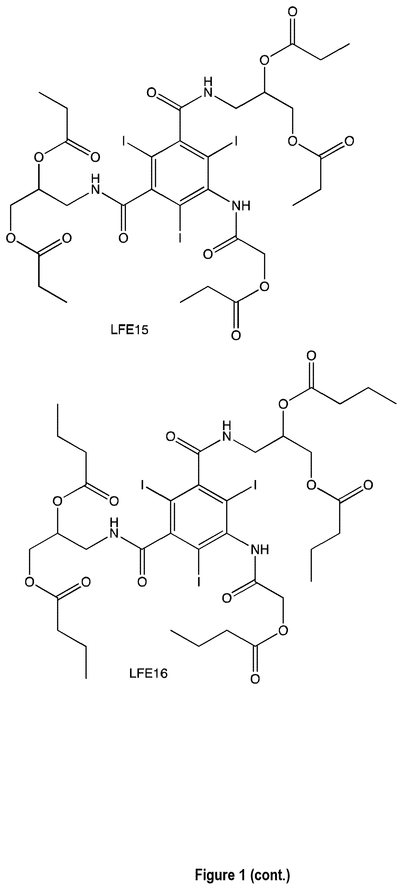

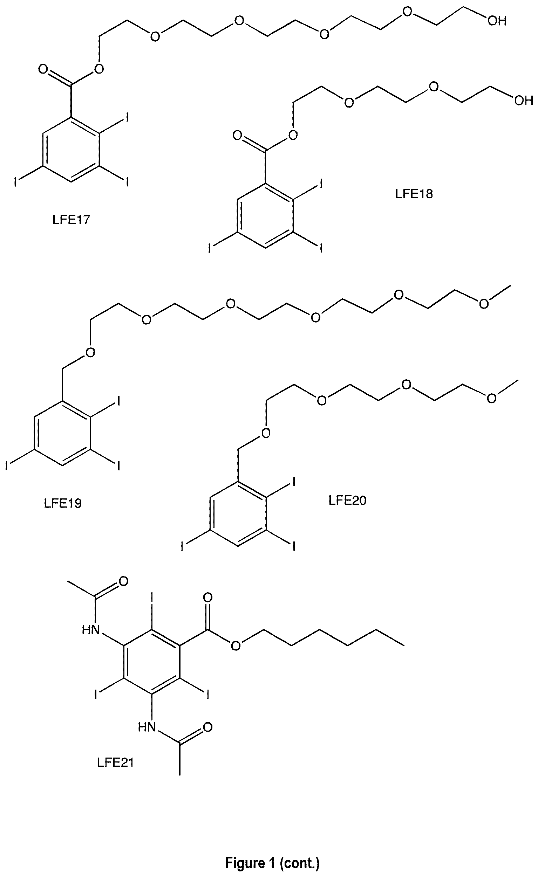

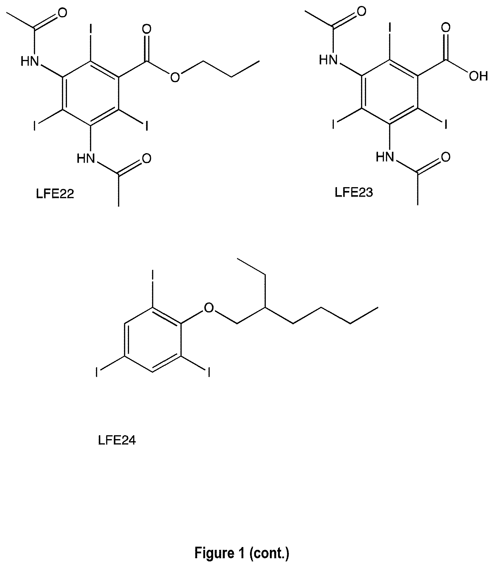

In particular embodiments of the first aspect of the invention, optionally in combination with any of the embodiments provided above or below, the radiopaque molecule is a molecule selected from the group consisting of LFE03, LFE04, LFE05, LFE06, LFE07, LFE08, LFE09, LFE10, LFE111, LFE12, LFE17, LFE18, LFE19, and LFE20 (as shown in figure 1). In more particular embodiments, the radiopaque molecule is a molecule selected from the group consisting of LFE05, LFE06, LFE07, LFE09, and LFE10 (as shown in figure 1).

In addition to the above, the radiopaque molecules considered for the present invention usually have a molecular weight that is equal or below 2500 g/mol. In particular embodiments of the first aspect of the invention, optionally in combination with any of the embodiments provided above or below, the radiopaque molecule has a molecular weight equal or below 2000 g/mol. In particular embodiments, the radiopaque molecule has a molecular weight equal or below 1900 g/mol, for example, 1800 g/mol, 1700 g/mol, 1600 g/mol, 1500 g/mol, 1400 g/mol, 1300 g/mol, 1200 g/mol, 1100 g/mol, 1000 g/mol, 900 g/mol, 800 g/mol, 700 g/mol, 600 g/mol, or 500 g/mol.

The present application also provides radiopaque molecules as defined above, as well as their use in embolization, for example, their use for providing radiopacity to embolizing compositions, or simply for preparing embolizing compositions. In particular embodiments, the application provides radiopaque molecules which are compounds with formula I, II, III, IV, or V, as defined above. In more particular embodiments, the invention provides a radiopaque molecule which is a molecule selected from the compounds of formula I to V that contains at least one pegylated chain. In other particular embodiments, the invention provides a radiopaque molecule which is a molecule selected from the group consisting of LFE03, LFE04, LFE05, LFE06, LFE07, LFE08, LFE09, LFE10, LFE111, LFE12, LFE17, LFE18, LFE19, and LFE20 (as shown in figure 1). In even more particular embodiments, the radiopaque molecule is a molecule selected from the group consisting of LFE05, LFE06, LFE07, LFE09, and LFE10 (as shown in figure 1).

### Polymers

The polymer in the liquid polymeric composition of the invention is one that is soluble in the non-physiological solution and insoluble in a physiological solution. When a polymer is soluble in solution, it can be easy to deploy through a delivery device, e.g. microcatheter, to a delivery site and/or treatment site. However, once precipitated out of solution, a polymer can be much more difficult to deploy. For example, once precipitated, a polymer can in some instances be more difficult to deploy through a delivery device. As such, the compositions and methods described herein can provide polymer treatment solutions to sites that would otherwise not be easily administered to without being soluble prior to exiting a delivery device. The polymer in the compositions of the invention is biocompatible.

The liquid polymeric compositions according to the first aspect of the invention comprise a non-physiological solvent or solvent system (e.g. a solvent having a non-physiological pH). The embolizing composition of the invention is thus a solution. The polymer is soluble in the solution but insoluble at physiological conditions. In some embodiments, the polymer can be soluble in a water-miscible organic solvent but insoluble at physiological conditions (e.g. water).

A function of the polymer, e.g. liquid embolic polymer, can be to precipitate when coming in contact with blood or other physiological fluid. Alternatively, if the solubility trigger is solubility in a water miscible organic solvent and insolubility at physiological conditions, any physiological environment can initiate the precipitation.

Precipitation of the polymer at physiological conditions can be used to occlude a biological structure. Control of the liquid embolic polymer's solubility can be achieved by selection of the composition of the polymer. In some embodiments of the first aspect of the invention, optionally in combination with any of the embodiments provided above or below, the polymers can be a reaction product of two different polymerizable monomers, three different polymerizable monomers, four different polymerizable monomers, five different polymerizable monomers, or more.

Polymerizable moieties can be those that permit free radical or anionic polymerization, including but not limited to ethylene, vinyl alcohol, vinyl, propylene, ethylene glycol, ethylene oxide, propylene oxide, acrylates, methacrylates, acrylamides, methacrylamides, and derivatives thereof. Alternatively, other reactive chemistries can be employed to polymerize the liquid embolic polymer, such as, but not limited to nucleophile/N-hydroxysuccinimde esters, nucleophile/halide, vinyl sulfone/acrylate, or maleimide/acrylate. Preferred polymerizable moieties can be ethylene, vinyl alcohol, acrylates and acrylamides.

In particular embodiments of the first aspect of the invention, optionally in combination with any of the embodiments provided above or below, the polymer is ethylene vinyl alcohol copolymer. Ethylene vinyl alcohol copolymer is a biocompatible polymer mainly composed of a random mixture of ethylene (hydrophobic) and vinyl alcohol (hydrophilic). This polymer is dissolved in a non-physiological solvent, for example, dimethyl sulfoxide (DMSO). When this mixture comes into contact with aqueous physiological media, e.g. blood, DMSO rapidly diffuses away, causing in situ precipitation and solidification of the polymer.

Ethylene vinyl alcohol copolymers used herein are either commercially available or can be prepared by art recognized procedures. In particular embodiments of the first aspect of the invention, optionally in combination with any of the embodiments provided above or below, the ethylene vinyl alcohol copolymer composition is selected such that a solution of 6 weight percent of the ethylene vinyl alcohol copolymer, together with 20-40 weight percent of a radiopaque molecule in DMSO has a viscosity equal to or less than 30 centistokes at 40°C. In particular embodiments of the first aspect of the invention, optionally in combination with any of the embodiments provided above or below, the ethylene vinyl alcohol copolymer has melt flow index ranging from 1 to 20. In other particular embodiments the ethylene vinyl alcohol copolymer has a melt flow index ranging from from 1,5 to 15, for example, from 2 to 5.

In particular embodiments of the first aspect of the invention, optionally in combination with any of the embodiments provided above or below, the ethylene vinyl alcohol copolymer comprises from about 25 to about 60 mole percent of ethylene and from about 40 to about 75 mole percent of vinyl alcohol. In particular embodiments of the first aspect of the invention, optionally in combination with any of the embodiments provided above or below, the ethylene vinyl alcohol copolymer comprises from about 30 to about 50 mole percent of ethylene and from about 50 to about 70 mole percent of vinyl alcohol. These compositions provide for appropriate precipitation rates suitable for use in embolizing blood vessels.

### Non-physiological solvent

The liquid polymeric composition according to the first aspect of the invention contains a non-physiological solvent or solvent system. A non-physiological solvent or solvent system has features that are non-physiological. Generally, the non-physiological solvent or solvent system is any solvent besides water. In some embodiments of the first aspect of the invention, optionally in combination with any of the embodiments provided above or below, the non-physiological solvent or solvent system is an organic solvent that is miscible with water.

According to the first aspect of the invention, the polymer and the radiopaque molecule are soluble in the non-physiological solvent or solvent system. The non-physiological solvent or solvent system in the compositions of the invention is also biocompatible at the concentrations used.

In one embodiment of the first aspect of the invention, optionally in combination with any of the embodiments provided above or below, the non-physiological solvent is dimethyl sulfoxide (DMSO), analogues/homologues of DMSO, dimethyl formamide, monohydric alcohols, polyhydric alcohols, ethoxylated polyhydric alcohols, methyl ethers of ethoxylated polyhydric alcohols, N-methylpyrrolidone, or combinations thereof. In other particular embodiments the non-physiological solvent is DMSO.

In some embodiments of the first aspect of the invention, optionally in combination with any of the embodiments provided above or below, the embolizing composition comprises a solvent system comprising more than one solvent. For example, the non-physiological solvent system may comprise one or more organic solvents that is/are miscible with water and water. In particular embodiments, the solvent system comprises dimethyl sulfoxide (DMSO), analogues/homologues of DMSO, dimethyl formamide, monohydric alcohols, polyhydric alcohols, ethoxylated polyhydric alcohols, methyl ethers of ethoxylated polyhydric alcohols, N-methylpyrrolidone, or combinations thereof. In particular embodiments, the solvent system comprises one of the solvents above or combinations thereof and water.

In particular embodiments, the non-physiological solvent system is alcohol and water. In other particular embodiments the non-physiological solvent is DMSO and water.

### Preparation method and products

A second aspect of the invention refers to a method for preparing a liquid polymeric composition as defined in the first aspect of the invention, the method comprising the steps of:
(i) providing a non-physiological solvent or solvent system, a polymer selected from the group consisting of ethylene vinyl alcohol copolymer, cellulose acetate, poly(2-hydroxyethylmethacrylate), copolymers of cellulose acetate, poly(2-hydroxyethylmethacrylate), or both, poly(ethylene oxide)-co-poly(propylene oxide) copolymers, poly(oligoethylene glycol)-(meth)acrylates, poly(meth)acrylate copolymers, poly(meth)acrylamide copolymers, and combinations thereof and a radiopaque compound and
(ii) mixing until a homogeneous dissolution is achieved.

All embodiments defined above for the solvent or solvent system, the polymer and the radiopaque molecule also apply for this second aspect of the invention.

In some embodiments of the second aspect of the invention, optionally in combination with any of the embodiments provided above or below, gentle heating and stirring can be used to effect dissolution of the copolymer into the biocompatible solvent, e.g., 12 hours at 50°C.

The particular order of addition of components to the solvent or solvent system is not critical and stirring of the resulting suspension is conducted as necessary to achieve homogeneity of the composition. Preferably, mixing/stirring of the composition is conducted under an anhydrous atmosphere at ambient pressure. The resulting composition may be heat sterilized and then stored. The resulting composition may be stored, for example, in sealed amber bottles or vials until needed.

The liquid polymeric composition can be removed from a vial using a needle and syringe, and the syringe can be later connected to a delivery device or catheter. Alternatively, the liquid embolic polymer formulation can be pre-packaged in a delivery syringe.

The invention thus provides in the third aspect an injectable (product) comprising a liquid polymeric composition as defined in the first aspect of the invention. All embodiments defined above for the solvent or solvent system, the polymer and the radiopaque molecule also apply for this third aspect of the invention. In some embodiments, the injectable is prepacked in a delivery syringe.

In a fourth aspect the invention also provides a kit for embolization, said kit comprising a container containing a liquid polymeric composition according to the first aspect of the invention. All embodiments defined above for the solvent or solvent system, the polymer and the radiopaque molecule also apply for this fourth aspect of the invention. In a particular embodiment of the fourth aspect of the invention, optionally in combination with any of the embodiments provided above or below, the container is a pre-packed delivery syringe or a vial. In another particular embodiment of this fourth aspect, the kit comprises further containers. Said further containers may contain, for example, a non-physiological solution for rinsing (rinsing solution). The rinsing solution may be the same as the solution in the embolizing composition. In more particular embodiments of this fourth aspect, the composition or the rinsing solution additionally contain a colored compound. The colored compound may be added in order to clearly differentiate the embolizing composition from the rinsing solution. In other particular embodiments the kit comprises instructions for using the embolizing composition.

### Medical indications

The compositions described above are then employed in methods for embolizing mammalian blood vessels. The fifth and sixth aspects of the invention respectively contemplate the compositions of the first aspect of the invention for use in embolizing a blood vessel and for use in filling a vascular defect.

All embodiments defined above for the solvent or solvent system, the polymer and the radiopaque molecule also apply for the fifth and sixth aspects of the invention.

In one embodiment of the fifth and sixth aspects of the invention, optionally in combination with any of the embodiments provided above or below, a sufficient amount of the composition according to the first aspect of the invention is introduced into the selected blood vessel by conventional means (e.g., injection or catheter delivery under fluoroscopy) so that upon precipitation of the polymer, the blood vessel is embolized. The particular amount of embolizing composition employed is dictated by the total volume of the vasculature to be embolized, the concentration of polymer in the composition, the rate of precipitation (solids formation) of the copolymer, etc. Such factors are well within the skill of the art. The rate of precipitation can be controlled by changing the overall hydrophobicity/hydrophilicity of the polymer with faster precipitation rates being achieved by a more hydrophobic polymer composition. This may be achieved, for example, by increasing the ethylene content in an ethylene vinyl alcohol copolymer in the embolizing compositions (when ethylene vinyl alcohol is the polymer).

In one embodiment of the fifth and sixth aspects of the invention, optionally in combination with any of the embodiments provided above or below, the embolizing composition according to the first aspect is delivered to the selected vascular site is via a small diameter medical catheter. The particular catheter employed is not critical provided that polymeric catheter components are compatible with the embolizing composition (i.e., the catheter components will not readily degrade in the embolizing composition). In this regard, in one particular embodiment polyethylene is used in the catheter components because of its inertness in the presence of the embolizing composition described herein. Other materials compatible with the embolizing compositions can be readily determined by the skilled artisan and include, for example, other polyolefins, fluoropolymers (e.g., Teflon, silicone, etc...).

When delivered by catheter, the injection rate dictates, in part, the form of the precipitate at the vascular site. Specifically, low injection rates of approximately 0.05 to 0.3 ml/minute will provide for a precipitate in the form of a kernel or nodule which is particularly beneficial for site specific embolization because the precipitate forms primarily at the point of injection. Contrarily, high injection rates of about 0.1 to 0.5 or more ml/several seconds (e.g., up to ten seconds) will provide for a filament like mass projecting downstream from the catheter tip which is particularly beneficial for providing the embolizing agent deep into the vascular tree. Such procedures are suitable for embolizing tumor masses, organs and arteriovenous malformations (AVM).

When introduced into the vascular site, the solvent diffuses rapidly into the blood and a solid precipitate forms which precipitate is the polymer with the radiopaque molecule encapsulated therein. Without being limited to any theory, it is believed that initially, a soft gel to spongy solid precipitate forms upon contact with the blood which precipitate is open and fibrous in structure. This precipitate then restricts blood flow, entrapping red cells thereby causing clot embolization of the blood vessel. The contrast agent encapsulated in the precipitate is slowly released or transformed by hydrolysis, enzymatic action or (de)protonation into a more water-soluble molecule, and consequently the precipitate loses radiopacity in a gradual manner. Eventually, the radiopacity vanishes completely and the embolized area becomes translucent to fluoroscopy. Gradual radiopacity loss may be advantageous since it may allow improved visualization in subsequent embolization procedures.

The embolizing of blood vessels as described above can be used in turn to prevent/control bleeding (e.g., organ bleeding, gastrointestinal bleeding, vascular bleeding, bleeding associated with an aneurysm) or to ablate diseased tissue (e.g., tumors, etc.). Thus, the invention also contemplates the compositions according to the first aspect of the invention for use in stopping bleeding, either partially or fully.

Additionally, the compositions defined above provide an appropriate vehicle for the delivery of a medicament to the vascular site. Thus, the invention also contemplates the compositions of the first aspect of the invention for use in the delivery of drugs. Specifically, a suitable medicament, e.g., a chemotherapeutic agent, growth factor agents, anti-inflammatory agents, anti-spasmatic agents, etc., which are compatible with the embolizing composition can be included in this composition in therapeutic levels and delivered directly to the vascular site.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### Examples

### Example 1: Synthesis of radiopaque compounds

### Synthesis of derivatives of diatrizoic acid: LFE21 and LFE22

The following example illustrates the synthesis of compound LFE21. Diatrizoic acid from Sigma Aldrich (LFE23, 1.2 equiv.), 1-bromohexane (1 equiv.), potassium carbonate (1.2 equiv.) and potassium iodide (1.2 equiv.) were dissolved in DMF and stirred overnight at 90°C. In the next morning, the reaction mixture was cooled down and precipitated in deionized water (10x volume of DMF). The precipitate was filtrated, washed with deionized water and dried under vacuum. The product was further purified by crystallization from methanol and hexane.

LFE22 was prepared following the same procedure as LFE23, except that the esterification of diatrizoic acid is performed with 1-bromopropane.

### Synthesis of derivatives of iomeprol: LFE14, LFE15 and LFE16

This example illustrates the synthesis of compound LFE14. Briefly, iomeprol was obtained from Bracco (LFE13, 1 equiv), acetic anhydride (25 quiv.) and anhydrous sodium bicarbonate (10 equiv.) were stirred in dry ethyl acetate at room temperature for 48h. After completion of the reaction, the mixture was filtered and the filtrate was concentrated. To the residue was added dichloromethane and water and the phases separated. The organic phase was dried (Na2SO4) and concentrated. The syrup was crystallized from a mixture of hexane and ethyl acetate.

LFE15 and LFE16 were prepared following the same procedure as LFE14, except that the esterification of iomeprol is performed with propionic and butyric anhydride respectively.

### Synthesis of derivatives of 3,5-diiodo-4-pyridone-N-acetic acid: LFE03 and LFE04

This example illustrates the synthesis of compound LFE03. Briefly, 3,5-diiodo-4-pyridone-N-acetic acid from Manchester Organics (1 part), 1-propanol (10 parts) and concentrated sulfuric acid (0.01 parts) were refluxed overnight. The resulting ester crystallized upon cooling. The product was recrystallized from methanol. LFE04 was prepared following the same procedure as LFE03, except that the esterification of 3,5-diiodo-4-pyridone-N-acetic acid is performed with 1-hexanol.

### Synthesis of derivatives of 2,4,6-triiodophenol: LFE01, LFE05, LFE06, LFE07, LFE08, LFE11, LFE12 and LFE24

This example illustrates the synthesis of compound LFE05. 2,4,6-triiodophenol from Sigma Aldrich (0.5 equiv.) **in** dry acetonitrile at 40 °C, potassium carbonate (2 equiv.) and potassium iodide (1 equiv.) was added and the reaction mixture was stirred for 1 h. 1-(2-Bromoethoxy)-2-(2-methoxyethoxy)ethane (1 equiv.) dissolved in acetonitrile was then added dropwise and the contents were heated at 80 °C overnight. After removal of the base by filtration the solvent was removed in vacuo and the resulting product was dissolved in ethyl acetate. The organic phase was washed with saturated NaHCO3 and water. The organic phase was dried over magnesium sulfate and filtered through a pad of silica. The eluted product was recrystallized from hexane and ethyl acetate.

LFE01, LFE06, LFE07, LFE08, LFE11, LFE12 and LFE24 were prepared following the same procedure as LFE05, , except that the etherification of 2,4,6-triiodophenol is performed with 1-bromohexane, 2-[2-(2-Chloroethoxy)ethoxy]ethanol, Br-Peg5-OH, Br-Peg12-OH, Br-Peg4-COOEt, Br-Peg3-OAc and 2-ethylhexyl bromide.

### Synthesis of derivatives of 2,3,5-triiodobenzoic acid: LFE17 and LFE18

The following example illustrates the synthesis of compound LFE18. 2,3,5-triiodobenzoic acid from Sigma Aldrich (1 equiv.) and triethylene glycol (1 equiv.) were dissolved in dichloromethane. Dicyclohexylcarbodiimide (1.1 equiv.) was dissolved separately in dichloromethane. Both solutions were cooled to -20°C and mixed together. A catalytic amount of 4-dimethylaminopyridine was added and the reaction mixture was kept at 4°C overnight. On the next morning, the reaction mixture was stirred at room temperature for 4h. The reaction mixture was filtered, and the organic phase was washed with saturated sodium bicarbonate several times and finally with water. The organic phase was dried over magnesium sulfate and rotavaporated. The product was recrystallized from hexane and ethyl acetate.

LFE17 was prepared following the same procedure as LFE18, except that the esterification of 2,3,5-triiodobenzoic acid is performed with pentaethylene glycol.

### Synthesis of derivatives of 2,4,6-triiodophenoxyacetic acid: LFE02, LFE09 and LFE10

The following example illustrates the synthesis of compound LFE09. Briefly, 2,4,6-triiodophenol from Sigma Aldrich (0.5 equiv.) in dry acetonitrile at 40 °C, potassium carbonate (2 equiv.) and potassium iodide (1 eqeiv.) was added and the reaction mixture was stirred for 1 h. Ethyl bromoacetate (1 equiv.) diluted in acetonitrile was then added dropwise and the contents were heated at 80 °C overnight. After removal of the base by filtration the solvent was removed in vacuo and the resulting product was dissolved in ethyl acetate. The organic phase was washed with saturated NaHCO3 and water. The organic phase was dried over magnesium sulfate and rotavaporated. This afforded ethyl 2,4,6-triiodophenoxyacetate, i.e. compound LFE02.

Ethyl 2,4,6-triiodophenoxyacetate (1 equiv.) and potassium hydroxide (4 equiv.) were dissolved in methanol and stirred overnight at room temperature. Methanol was evaporated and the solid was dissolved in water. The mixture was acidified with HCl 2N and the resulting precipitate was filtered, washed with water and dried to afford 2,4,6-triiodophenoxyacetic acid.

2,4,6-triiodophenoxyacetic acid (1 equiv.) and triethylene glycol monomethyl ether (1 equiv.) were dissolved in dichloromethane. Dicyclohexylcarbodiimide (1.1 equiv.) was dissolved separately in dichloromethane. Both solutions were cooled to -20°C and mixed together. A catalytic amount of 4-dimethylaminopyridine was added and the reaction mixture was kept at 4°C overnight. On the next morning, the reaction mixture was stirred at room temperature for 4h. The reaction mixture was filtered, and the organic phase was washed with saturated sodium bicarbonate several times and finally with water. The organic phase was dried over magnesium sulfate and rotavaporated. The product was recrystallized from hexane and ethyl acetate.

LFE10 was prepared following the same procedure as LFE09, except that the esterification of 2,4,6-triiodophenoxyacetic acid is performed with triethylene glycol.

### Synthesis of 2,3,5-triiodobenzyl derivatives: LFE19 and LFE20

The following example illustrates the synthesis of compound LFE20. Briefly, to an ice cooled 2,3,5-triiodobenzyl from Sigma Aldrich (1.0 equiv.) alcohol solution in ether, PBr3 (1.5 equiv.) was added dropwise under nitrogen. The mixture was stirred at room temperature overnight and the reaction was quenched by the addition of H₂O (15 mL) in small portions at 0°C. The aqueous phase was removed, and the organic layer was washed with H₂O, dried over MgSO4 and evaporated *in vacuo.* Purification of 2,3,5-triiodobenzyl bromide was achieved by silica gel chromatography using the hexane and ethyl acetate as solvent.

To an ice cooled solution of triethylene glycol monomethyl ether (1.0 equiv.) in THF, sodium hydride (60% in mineral oil dispersion, 1.04 equiv.) was added slowly. The mixture was stirred for 30 min at room temperature, after which 2,3,5-triiodobenzyl bromide (1 equiv.) dissolved in THF was added dropwise. The reaction was stirred overnight at room temperature and then quenched with H₂O. The product was extracted four times with ethyl acetate and the combined organic phases were dried with MgSO₄ and rotavaporated. The product was purified by silica gel chromatography using hexane and ethyl acetate as eluent.

LFE19 was prepared following the same procedure as LFE20, except that the etherification of 2,3,5-triiodobenzyl bromide is performed with pentaethylene glycol monomethyl ether.

### Example 2: Compositions containing polymer and contrast agents. LogP of contrast agents and viscosity and precipitation behavior of the compositions

The radiopaque compounds of example 1 were dissolved at a concentration of 150 mg equivalent iodine/ml together with ethylene vinyl alcohol copolymer (44% ethylene) (Sigma Aldrich 414107) dissolved at 6% in DSMO. The resulting solutions were assayed to assess the precipitation behavior and the radiopacity loss. Viscosity of the resulting solutions was determined using a rheometer with a parallel plate configuration (Anton Paar MCR92). Measurements were performed on freshly prepared solutions with a 100-mm diameter plate at 40°C. Embolization formulations had viscosities ranging from 20 to 35 cst at 40°C.

The logP of the synthesized contrast agents was calculated using MarvinSuite software (ChemAxon, version 20.7.0). The calculation method is based on the publication of Viswanadhan *et al* (J. Chem. Inf Comput. Sci. 1989, 29, 163-172).

Embolization formulations were slowly injected into phosphate buffered saline (PBS) using a syringe and a fine needle (27 G). Precipitates of embolizing material were obtained after slow injection (1 minute approximately) of 100 ul of embolization formulations. The precipitation behavior was observed and scored as cohesive (2), cohesive with loss of some material (1) and uncontrollable injection or not cohesive (0).

Radiopacity loss of precipitates was determined by comparing the radiopacity of freshly prepared samples (time = 0) and samples incubated for 30 days in phosphate buffered saline (PBS) at 37°C. PBS was changed daily when possible. Radiopacity was determined using a C-arm X-ray equipment (OEC Fluorostar 7900 COMPACT2, General Electric). Radiopacity loss was scored as radiostable (score = 0) when the radiopacity loss was below 5%, slightly radiolabile when the radiopacity loss was comprised between 5 to 25% radiopacity loss (score = 1), moderately radiolabile when the radiopacity loss was comprised between 26 to 69% radiopacity loss (score = 2) and highly radiolabile when the radiopacity loss was comprised between 70 to 100% radiopacity loss (score = 3). Homogeneity of the radiopacity was also scored as non-homegeneous (score = 0) or homogeneous (score = 1).

**Table 1. LogP, precipitation/cohesive behavior and radiopacity loss of different iodinated compounds.**

| Compound | Molecular weight (g/mol) | % iodine per molecule (wt%) | LogP | Radiopacity loss (0-3) | Precipitation (0-2) | Homogeneous radiopacity (0-1) | Total score |
|---|---|---|---|---|---|---|---|
| LFE01 | 556 | 68.5 | 6.8 | 1 | 2 | 0 | 3 |
| LFE02 | 558 | 68.3 | 4.7 | 2 | 1 | 0 | 3 |
| LFE03 | 447 | 56.8 | 3.9 | 3 | 2 | 1 | 6 |
| LFE04 | 517 | 49.1 | 5.1 | 2 | 2 | 1 | 5 |
| LFE05 | 618 | 61.7 | 4.3 | 3 | 2 | 0 | 5 |
| LFE06 | 603 | 63.2 | 3.7 | 2 | 2 | 1 | 5 |
| LFE07 | 706 | 54.0 | 3.9 | 2 | 2 | 1 | 5 |
| LFE08 | 1014 | 37.6 | 2.8 | 2 | 0 | 1 | 3 |
| LFE09 | 676 | 56.4 | 3.9 | 3 | 2 | 1 | 6 |
| LFE10 | 662 | 57.6 | 3.3 | 3 | 2 | 1 | 6 |
| LFE11 | 646 | 59.0 | 4.4 | 2 | 2 | 0 | 4 |
| LFE12 | 646 | 59.0 | 4.2 | 2 | 2 | 0 | 4 |
| LFE13 | 777 | 49.0 | -1.6 | 3 | 0 | 1 | 4 |
| LFE14 | 987 | 38.6 | 0.6 | 3 | 2 | 1 | 6 |
| LFE15 | 1127 | 33.8 | 5.7 | 1 | 2 | 1 | 4 |
| LFE16 | 1057 | 36.0 | 3.7 | 2 | 2 | 1 | 5 |
| LFE17 | 720 | 52.9 | 3.8 | 2 | 2 | 1 | 5 |
| LFE18 | 632 | 60.3 | 4.1 | 2 | 2 | 1 | 5 |
| LFE19 | 632 | 60.3 | 3.9 | 1 | 2 | 1 | 4 |
| LFE20 | 720 | 52.9 | 4.3 | 1 | 2 | 0 | 3 |
| LFE21 | 698 | 54.6 | 5.8 | 1 | 2 | 1 | 4 |
| LFE22 | 656 | 58.1 | 4.6 | 1 | 2 | 1 | 4 |
| LFE23 | 614 | 62.1 | 3.2 | 3 | 1 | 1 | 5 |
| LFE24 | 584 | 65.2 | 7.6 | 0 | 1 | 0 | 1 |

Embolization compounds with higher scores for cohesiveness and radiopacity loss were prioritized as potential candidates for preparing embolizing formulations with programmed radiopacity loss.

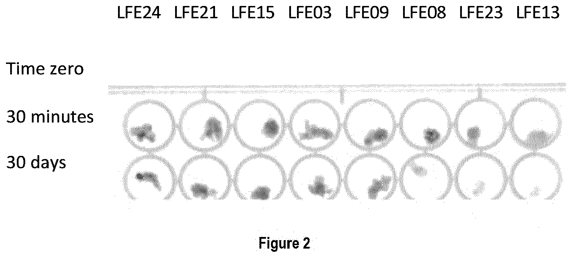

Figure 2 shows the radiopacity loss in PBS over time (at time 0, and after incubating for 30 minutes and 30 days at 37°C) of representative compounds described above. The following radiopaque compounds having logP values ranging from -1,6 to 7,6 were assayed: LFE24, LFE21, LFE15, LFE03, LFE09, LFE08, LFE23 and LFE13. The procedure was as defined above. It can be observed that formulations with high logP values, i.e. above 7.0, are radiostable, while formulations with low logP values, i.e. below 2.0 (logP(LFE13) = -1.6 and logP(LFE23) at pH 7.4 = -0.5) lose their radiopacity within minutes. However, formulations with compound with intermediate logP values, comprised between 2.8 and 5.8, maintain substantial radiopacity for the first 30 min, while showing significant radiopacity loss after incubation in PBS for 30 days at 37°C.

Quantification of radiopacity is shown in table 2. Compound LFE24, with a logP = 7.6, showed complete radiostability after 30 days in PBS at 37°C. Formulations prepared with radiopaque compounds LFE03, LFE08, LFE09, LFE15 and LFE21, with logP values comprised between 2.8 and 5.8, preserved more than 50% of their radiopacity after 30 min, but showed significant radiopacity loss after 30 days in PBS at 37°C.

The radiopacity loss correlated positively with the logP values of these compounds, suggesting that compounds in this range of logP values may be appropriate for embolization purposes where the radiopaque compound may need to be bioresorbed in less than 6 months. Contrarily, formulations prepared with highly water-soluble radiopaque compounds, such as LFE13 and LFE23, which had a logP in PBS of -1.6 and -0.5 respectively, showed a loss of radiopacity greater than 50% of their radiopacity at time zero after 30 minutes, suggesting that their water solubility and diffusion may be too fast and may not allow correct monitoring of the embolization process.

**Table 2. Quantification of radiopacity loss of different formulations after 30 min and 30 days of incubation in PBS at 37°C.**

| | **% of radiopacity compared to time zero** | | | | | | | |
|---|---|---|---|---|---|---|---|---|
| | **LFE24** | **LFE21** | **LFE15** | **LFE03** | **LFE09** | **LFE08** | **LFE23** | **LFE13** |
| **t zero** | 100.0 | 100.0 | 100.0 | 100.0 | 100.0 | 100.0 | 100.0 | 100.0 |
| **30 min** | 101.3 | 98.3 | 94.5 | 100.9 | 83.9 | 54.2 | 35.6 | 29.0 |
| **30 days** | 101.9 | 91.4 | 85.5 | 14.5 | 64.7 | 29.3 | 19.0 | 11.9 |

### Example 3: In vivo determination of radiopacity loss

Embolizing formulations containing different compounds described in example 2 were evaluated *in vivo* for radiopacity loss at 150 mg eq iodine/ml. First, formulations were precipitated in 2% agarose molds and incubated for 2 hours at room temperature to ensure formation of rod-like precipitates. Subsequently, the resulting rods were carefully removed from molds and incubated for 1 hour in deionized water with mild shaking to remove dimethyl sulfoxide. The resulting rods were allowed to dry overnight under mild vacuum and charged into pet microchip syringes. Microchip syringes charged with rod-like precipitates were sterilized by ethylene oxide and resulting precipitates ultimately implanted subdermally in a New Zealand rabbit (4.5 Kg). The radiopacity of the implants was determined by X-Ray imaging right after implantation and after 4 weeks. After 4 weeks, the implants were removed and their radiopacity was also determined *ex vivo.*

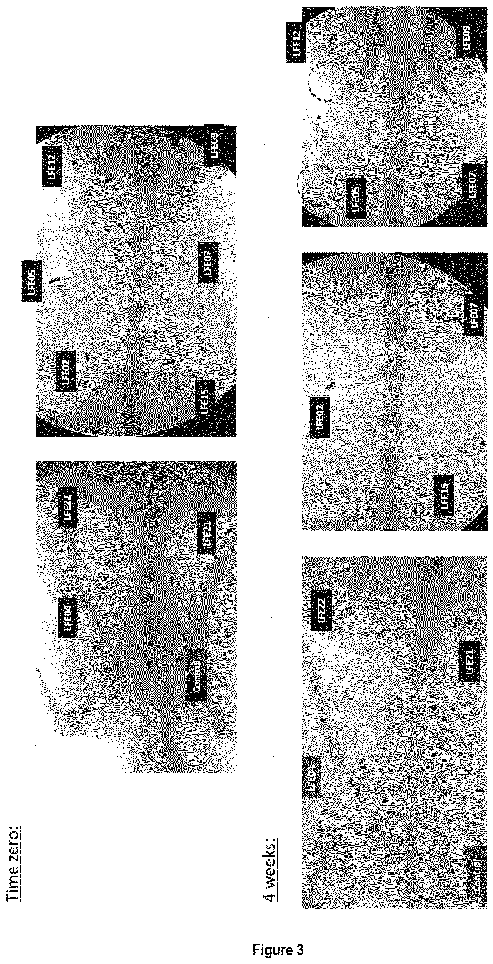

Results, shown in figure 3, show that radiopacity was gradually lost over the course of 4 weeks. For some implants, i.e. LFE05, LFE07, LFE09 and LFE12, radiopacity was sufficiently retained for/at the initial hours, however radiopacity loss was complete or almost complete after 4 weeks of implantation. Radiopacity of these implants was not observed when monitored under X-ray fluoroscopy, suggesting that these contrast agents were fully bioresorbed and are good candidates for embolization purposes. The rest of implants, LFE04, LFE22, LFE21, LFE02 and LFE15, could be clearly localized under fluoroscopy after 4 weeks of implantation. However, different degrees of radiopacity loss were observed, suggesting that the different radiopaque molecules were bioresorbed at different rates, accordingly with their physicochemical properties. The rate of radiopacity loss was compatible with a loss greater than 50% after 6 months, suggesting that these compounds with logP comprised between 2.0 and 7.0 are also good candidates for embolization purposes. Control implant, which consisted of a steel wire covered with ethylene vinyl alcohol copolymer, showed no changes in radiopacity after 4 weeks of implantation.

Radiopacity loss was quantified *ex vivo* by comparing the radiopacity of retrieved implants and control implants that had not been implanted *in vivo.* Results show a positive correlation between radiopacity loss and the partition coefficient (logP) of radiopaque materials, as shown in table 3.

**Table 3. Determination of radiopacity loss of implants after 4 weeks of implantation**

| **Compound** | **logP** | **Radiopacity loss (%)** |
|---|---|---|
| Control | - | 3.6 |
| LFE04 | 5.1 | 15.3 |
| LFE21 | 5.8 | 11.2 |
| LFE22 | 4.6 | 23.8 |
| LFE15 | 5.7 | 9.3 |
| LFE02 | 4.7 | 27.5 |
| LFE05 | 4.3 | 97.9 |
| LFE07 | 3.9 | 94.8 |
| LFE12 | 4.2 | 92.8 |
| LFE09 | 3.9 | 87.1 |

### Example 4: In vivo evaluation of the embolization formulation LFE09 in porcine kidney

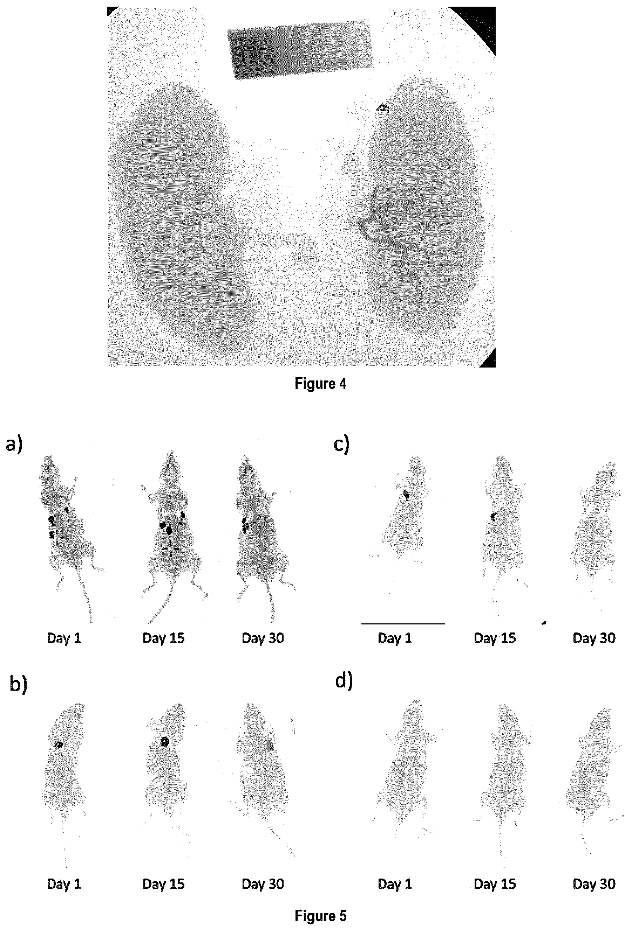

In this example, a 35 kg swine was prepared for blood vessel embolization using the embolic composition LFE09 prepared according to Example 2. Embolization of the left kidney proceeded by placement of a microcatheter into the kidney. The catheter was advanced into the renal artery, flushed with contrast agent to identify the location and then flushed with DMSO, followed by 0.6 ml of the LFE09 composition described above, followed yet by more DMSO within the catheter. LFE09 composition was quickly injected into the renal artery. After delivery of the composition, the upper pole of the kidney was blocked. After 14 days, the right kidney was embolized with 0.6 ml of the same LFE09 formulation and the animal was euthanized. Radiopacity was compared in both kidneys as shown in figure 4. Results indicate that the compositions of this invention are suitable for *in vivo* embolization of blood vessels in mammalian subjects and that radiopacity is gradually lost.

### Example 5: Radiopacity loss vs logP of radiopaque molecules in vivo

Embolization formulations were prepared by dissolving the appropriate radiopaque molecule at a concentration of 150 mg equivalent iodine/ml together with ethylene vinyl alcohol copolymer (44% ethylene) (Sigma Aldrich 414107) dissolved at 6% in DSMO. In this example, LFE01 (logP 6.8), LFE04 (logP 3.9) and iomeprol (logP -2.8) were used. As a control, an embolization formulation was prepared with 300 mg/ml of micronized Tantalum together with ethylene vinyl alcohol copolymer (44% ethylene) (Sigma Aldrich 414107) dissolved at 6% in DSMO. However, in this case a suspension was obtained. The resulting formulations were injected subdermally in mice using an insuline syringe (30 microlitres per mouse, 3 animals per group of radiopaque material). Mice were euthanized at day 1 (5 minutes after injection of the embolization formulation), day 15 and day 30. The formation and presence of the radiopaque precipitate was monitored using an OEC C-arm fluoroscopic equipment (GE). The radiopacity loss, i.e. the release of the radiopaque material from the resulting radiopaque precipitates, was assessed using the same equipment following the standard method ASTM F640-20 Standard Test Methods for Determining Radiopacity for Medical Use. The radiopacity loss was expressed as the percentage of remaining radiopacity compared to day 1, i.e. the radiopacity of the radiopaque precipitate formed 5 minutes after injection. The results of this example can be found in the following table:

| | **Remaining radiopacity (%)** | | |
|---|---|---|---|
| **Radiopaque material** | day 1 | day 15 | day 30 |
| **Tantalum** | 100,0 | 95,4 | 110,6 |
| **LFE01 (logP 6,8)** | 100,0 | 93,3 | 84,5 |
| **LFE04 (logP 3,9)** | 100,0 | 49,5 | 3,7 |
| **lomeprol (logP -2,8)** | 100,0 | -2,0 | 2,0 |

Embolization formulations with Tantalum showed constant radiopacity throughout the whole experiment, confirming that Tantalum was not released from the radiopaque precipitate (figure 5a). Embolization formulations with iodine-containing radiopaque molecules showed increasing radiopacity loss with decreasing logP values. Embolization formulation with LFE01 compound, which is a radiopaque molecule with logP 6.8, showed a cohesive precipitate which released part of the radiopaque molecule after 30 days (figure 5b).

Embolization formulation with LFE04 compound, which is a radiopaque molecule with a moderate logP value (logP 3.9), showed a precipitate with homogeneous radiopacity and the radiopaque molecule was completely released after 30 days (figure 5c). In contrast, embolization formulation with iomeprol, which is a water-soluble contrast agent with a low logP value (logP -2.8), showed a non-cohesive precipitate with a rapidly disappearing radiopacity, which was hardly visible after 5 minutes of injection due to rapid dissolution after injection (figure 5d). Therefore, appropriate selection of logP parameter may render a cohesive precipitate with homogeneous radiopacity suitable for embolization applications, with the advantage of complete radiopacity loss within a medically convenient time frame.

### Citation List

Viswanadhan et al. J. Chem. Inf Comput. Sci. 1989, 29, 163-172

## Claims

1. A liquid polymeric composition comprising:
(a) from about 2 to about 20 weight percent of a polymer selected from the group consisting of ethylene vinyl alcohol copolymer, cellulose acetate, poly(2-hydroxyethylmethacrylate), copolymers of cellulose acetate, poly(2-hydroxyethylmethacrylate), or both, poly(ethylene oxide)-co-poly(propylene oxide) copolymers, poly(oligoethylene glycol)-(meth)acrylates, poly(meth)acrylate copolymers, poly(meth)acrylamide copolymers, and combinations thereof;
(b) from about 5 to about 40 weight percent of a radiopaque molecule with a molecular weight below 2000 g/mol; and
(c) from about 40 to about 93 weight percent of a non-physiological solvent or solvent system;
wherein:
(i) the sum of the weight percent of all components in the composition is 100,
(ii) the polymer and the radiopaque molecule are not covalently bound and are both dissolved in the non-physiological solvent or solvent system,
(iii) the polymer is soluble in the non-physiological solution and insoluble in a physiological solution at room temperature, and
(iv) the liquid polymeric composition is capable of forming, upon contact with physiological conditions, a radiopaque precipitate which maintains at least 50% of its radiopacity during at least 30 minutes and loses at least 50% of its radiopacity in less than 6 months, in particular, the precipitate maintains at least 50% of its radiopacity during at least 1 hour and loses at least 50% of its radiopacity in less than 3 months.

2. The liquid polymeric composition according to claim 1, wherein the liquid polymeric composition forms a precipitate with homogenous radiopacity upon contact with physiological conditions.

3. The liquid polymeric composition according to any one of claims 1-2, wherein the radiopaque compound is an aromatic molecule which in the composition has a LogP from 2.0 to 9.0.

4. The liquid polymeric composition according to any one of claims 1-3, wherein the radiopaque compound is a non-ionizable aromatic molecule which in the composition has a LogP from 2.0 to 7.0, in particular from 3.0 to 5.0, or an ionizable aromatic molecule which in the composition has a LogP from 4.0 to 9.0, in particular from 5.0 to 8.0, and comprises acidic or basic groups.

5. The liquid polymeric composition according to any one of claims 1-4, wherein the radiopaque molecule is an aromatic molecule containing iodine or bromine at concentration greater than 300 mg equivalent of iodine or bromine per gram of molecule.

6. The liquid polymeric composition according to any one of claims 1-5, wherein the radiopaque molecule is a pegylated derivative of triiodophenol, iophenoxic acid, triiodobenzoic acid, 3,5-diiodo-4-pyridone diatrizoic acid, 5-amino-2,4,6-triiodoisophthalic acid or triiodoaniline.

7. The liquid polymeric composition according to any one of claims 1-6, wherein the radiopaque molecule is a compound of Formula I or Formula II 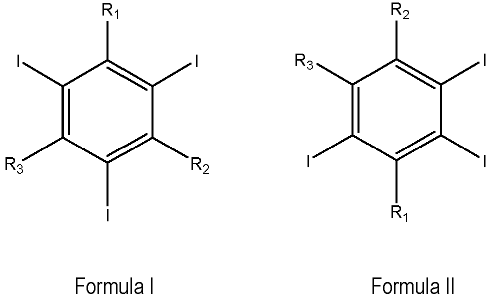 wherein:
R2 and R3 are independently selected from the group consisting of -H, -COOH, -(C₁-C₂₅)alkyl, -(C₁-C₂₅)alkyl-COOH, -O-(C₁-C₂₅)alkyl, -(C₁-C₂₅)alkyl-COO-(C₁-C₂₅)alkyl, -O-(CH₂-CH₂-O)_{b}-CH₃, -NH-CO-(C₁-C₂₅)alkyl, - NH₂, -NH-(C₁-C₂₅)alkyl, -N-((C₁-C₁₀)alkyl)₂ -CF₃, -Cl, -F, -CN, -NO₂, and -CO-NH-(C₁-C₂₅)alkyl, and
R1 is independently selected from the group consisting of:
-X-(C₁-C₂₀)alkyl-COO-R4
-X-(CH₂-CH₂-O)_{b}-R4,
-X-(CH₂-CH₂-O)_{b}-(CH₂)_{c}-COO-R4,
-X-(CH₂-CH₂-O)_{b}-COO-R4,
-X-(C₁-C₁₀)alkyl-COO-(CH₂-CH₂-O)_{b}-R4,
-X-(C₁-C₂₀)alkyl-NH₂,
-X-(C₁-C₁₀)alkyl-NH-(C₁-C₁₀)alkyl
-X-(C₁-C₁₀)alkyl-N-((C₁-C₁₀)alkyl)2
-(CH₂)_{c}-O-(CH₂-CH₂-O)_{b}-R4,
wherein
X is independently selected from the group consisting of O, COO and C(O)NH-R4 is selected from the group consisting of -H, -OH, -(C₁-C₈)alkyl, -(C₁-C₈)alkyl-OH, (C₁-C₈)acyl, -(CH₂-CH₂-O)_{b}-CH₃, and an alkyl chain containing at least one linkage capable of being chemically or enzymatically hydrolyzed in a physiological environment,
b is an integer from 0 to 20, and
c is an integer from 0 to 10.
or alternatively, the radiopaque molecule is a compound of Formula V: 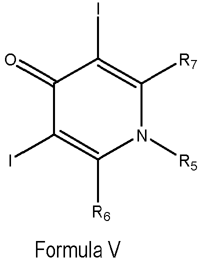
wherein,
R6 and R7 are independently selected from the group consisting of -H, -COOH, -(C₁-C₂₅)alkyl, -(C₁-C₂₅)alkyl-COOH, -O-(C₁-C₂₅)alkyl, -(C₁-C₂₅)alkyl-COO-(C₁-C₂₅)alkyl, -O-(CH₂-CH₂-O)_{b}-CH₃,-NH-CO-(C₁-C₂₅)alkyl, -NH₂, -NH-(C₁-C₂₅)alkyl, -N-((C₁-C₁₀)alkyl)₂ -CF₃, -Cl, -F, -CN, -NO₂, and -CO-NH-(C₁-C₂₅)alkyl, and
R5 is -(CH₂)_{d}-COO-R8, wherein d is an integer from 0 to 10 and R8 is selected from the group consisting of - H, -OH, -(C₁-C₈)alkyl, -(C₁-C₈)alkyl-OH, (C₁-C₈)acyl, -(CH₂-CH₂-O)_{b}-CH₃, wherein b is an integer from 0 to 20, and an alkyl chain containing at least one linkage capable of being chemically or enzymatically hydrolyzed in a physiological environment.

8. The liquid polymeric composition according to claim 6, wherein the radiopaque molecule is selected from a compound of formula III and a compound of formula IV: 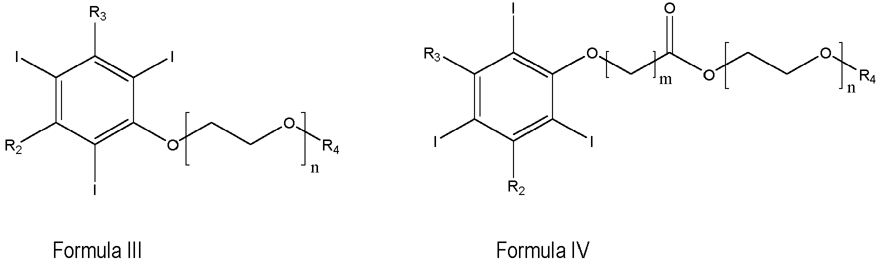 Wherein,
n and m are independently an integer from 1 to 20
R2 and R3 are independently selected from the group consisting of -H, -COOH, -(C₁-C₂₅)alkyl, -(C₁-C₂₅)alkyl-COOH, -O-(C₁-C₂₅)alkyl, -(C₁-C₂₅)alkyl-COO-(C₁-C₂₅)alkyl, -O-(CH₂-CH₂-O)_{b}-CH₃,-NH-CO-(C₁-C₂₅)alkyl, -NH₂, -NH-(C₁-C₂₅)alkyl, -N-((C₁-C₁₀)alkyl)₂ -CF₃, -Cl, -F, -CN, -NO₂, and -CO-NH-(C₁-C₂₅)alkyl, and
R4 is selected from the group consisting of from -H, -OH, -(C₁-C₈)alkyl, -(C₁-C₈)alkyl-OH, (C₁-C₈)acyl, -(CH₂-CH₂-O)b-CH₃, wherein b is an integer from 0 to 20, and an alkyl chain containing at least one linkage capable of being chemically or enzymatically hydrolyzed in a physiological environment.

9. The liquid polymeric composition according to any one of claims 1-8, wherein the radiopaque molecule contains a terminal hydroxyl.

10. The liquid polymeric composition according to any of the claims 7-9, wherein the at least one linkage capable of being chemically or enzymatically hydrolyzed in a physiological environment is selected from the group consisting of an ester, a polyester, an amide, and a carbonate.

11. The liquid polymeric composition according to any of the claims 1-10, wherein the non-physiological solvent or solvent system comprises an organic solvent that is miscible with water, in particular, wherein the non-physiological solvent or solvent system comprises dimethyl sulfoxide, dimethyl formamide, monohydric alcohols, polyhydric alcohols, ethoxylated polyhydric alcohols, N-methylpyrrolidone, methyl ethers of ethoxylated polyhydric alcohols, or combinations thereof, in particular, the non-physiological solvent or solvent system comprises dimethyl sulfoxide.

12. The liquid polymeric composition according to any of the claims 1-11, wherein the viscosity of the composition is below 50 cSt at 40°C.

13. An injectable comprising a liquid polymeric composition as defined in any of the claims 1-12.

14. A liquid polymeric composition as defined in any of the claims 1-12 or an injectable as defined in claim 13 for use in embolizing a blood vessel.

## Patentansprüche

1. Eine flüssige polymere Zusammensetzung umfassend:
(a) von etwa 2 bis etwa 20 Gew.- % eines Polymers, das ausgewählt ist aus der Gruppe bestehend aus Ethylen-Vinylalkohol-Copolymer, Celluloseacetat, Poly(2-hydroxyethylmethacrylat), Copolymeren von Celluloseacetat, Poly(2-hydroxyethylmethacrylat), oder beiden, Poly(ethylenoxid)-co-poly(propylenoxid)-Copolymeren, Poly(oligoethylenglykol)-(meth)acrylaten, Poly(meth)acrylat-Copolymeren, Poly(meth)acrylamid-Copolymeren und Kombinationen davon;
(b) von etwa 5 bis etwa 40 Gew.- % eines röntgendichten Moleküls mit einem Molekulargewicht unter 2000 g/mol; und
(c) von etwa 40 bis etwa 93 Gew.- % eines nicht-physiologischen Lösungsmittels oder Lösungsmittelsystems;
wobei:
(i) die Summe der Gew.-% aller Komponenten in der Zusammensetzung 100 beträgt,
(ii) das Polymer und das röntgendichte Molekül nicht kovalent gebunden sind und beide in dem nicht-physiologischen Lösungsmittel oder Lösungsmittelsystem gelöst sind,
(iii) das Polymer in der nicht-physiologischen Lösung löslich und in einer physiologischen Lösung bei Raumtemperatur unlöslich ist und
(iv) die flüssige polymere Zusammensetzung in der Lage ist, bei Kontakt mit physiologischen Bedingungen einen röntgendichten Niederschlag zu bilden, der mindestens 50 % seiner Röntgendichtkeit während mindestens 30 Minuten beibehält und mindestens 50 % seiner Röntgendichtkeit in weniger als 6 Monaten verliert, insbesondere der Niederschlag mindestens 50 % seiner Röntgendichtkeit während mindestens 1 Stunde beibehält und mindestens 50 % seiner Röntgendichtkeit in weniger als 3 Monaten verliert.

2. Die flüssige polymere Zusammensetzung nach Anspruch 1, wobei die flüssige polymere Zusammensetzung bei Kontakt mit physiologischen Bedingungen einen Niederschlag mit homogener Röntgendichtkeit bildet.

3. Die flüssige polymere Zusammensetzung nach einem der Ansprüche 1 bis 2, wobei die röntgendichte Verbindung ein aromatisches Molekül ist, das in der Zusammensetzung ein LogP von 2,0 bis 9,0 hat.

4. Die flüssige polymere Zusammensetzung nach einem der Ansprüche 1 bis 3, wobei die röntgendichte Verbindung ein nichtionisierbares aromatisches Molekül ist, welches in der Zusammensetzung einen LogP von 2,0 bis 7,0, insbesondere von 3,0 bis 5,0, hat, oder ein ionisierbares aromatisches Molekül ist, welches in der Zusammensetzung einen LogP von 4,0 bis 9,0, insbesondere von 5,0 bis 8,0, hat und saure oder basische Gruppen umfasst.

5. Die flüssige polymere Zusammensetzung nach einem der Ansprüche 1 bis 4, wobei das röntgendichte Molekül ein aromatisches Molekül ist, das Jod oder Brom in einer Konzentration von mehr als 300 mg Äquivalent Jod oder Brom pro Gramm Molekül enthält.

6. Die flüssige polymere Zusammensetzung nach einem der Ansprüche 1 bis 5, wobei das röntgendichte Molekül ein pegyliertes Derivat von Triiodphenol, lophenoxinsäure, Triiodbenzoesäure, 3,5-Diiod-4-pyridondiatrizonsäure, 5-Amino-2,4,6-triiodisophthalsäure oder Triiodanilin ist.

7. Die flüssige polymere Zusammensetzung nach einem der Ansprüche 1 bis 6, wobei das röntgendichte Molekül eine Verbindung der Formel I oder Formel II ist 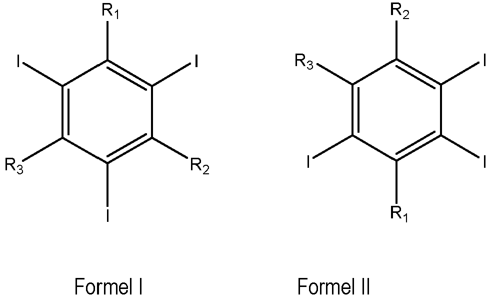 wobei:
R2 und R3 unabhängig voneinander ausgewählt sind aus der Gruppe bestehend aus -H, -COOH, -(C₁-C₂₅)-Alkyl, -(C₁-C₂₅)-Alkyl-COOH, -O-(C₁-C₂₅)-Alkyl, -(C₁-C₂₅)-Alkyl-COO-(C₁-C₂₅)-Alkyl, -O-(CH₂-CH₂-O)_{b}-CH₃, - NH-CO-(C₁-C₂₅)-Alkyl, -NH₂, -NH-(C₁-C₂₅)-Alkyl, -N-((C₁-C₁₀)-Alkyl)₂ -CF₃, -Cl, -F, -CN, -NO₂ und -CO-NH-(C₁-C₂₅)-Alkyl, und
R1 unabhängig ausgewählt ist aus der Gruppe bestehend aus:
-X-(C₁-C₂₀)Alkyl-COO-R4
-X-(CH₂-CH₂-O)_{b}-R4,
-X-(CH₂-CH₂-O)_{b}-(CH₂)_{c}-COO-R4,
-X-(CH₂-CH₂-O)_{b}-COO-R4,
-X-(C₁-C₁₀)Alkyl-COO-(CH₂-CH₂-O)_{b}-R4,
-X-(C₁-C₂₀)Alkyl-NH₂,
-X-(C₁-C₁₀)Alkyl-NH-(C₁-C₁₀)Alkyl
-X-(C₁-C₁₀)Alkyl-N-((C₁-C₁₀)Alkyl)2
-(CH₂)_{c}-O-(CH₂-CH₂-O)_{b}-R4,
wobei
X unabhängig ausgewählt ist aus der Gruppe bestehend aus O, COO und C(O)NH-R4 ausgewählt ist aus der Gruppe bestehend aus -H, -OH, -(C₁-C₈)-Alkyl, -(C₁-C₈)-Alkyl-OH, (C₁-C₈)-Acyl, - (CH₂-CH₂-O)_{b}-CH₃ und einer Alkylkette, die mindestens eine Verknüpfung enthält, die in einer physiologischen Umgebung chemisch oder enzymatisch hydrolysiert werden kann,
b eine ganze Zahl von 0 bis 20 ist, und
c eine ganze Zahl von 0 bis 10 ist.
oder, ersatzweise, ist das röntgendichte Molekül eine Verbindung der Formel V: 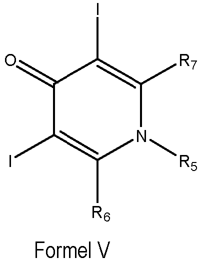
wobei,
R6 und R7 unabhängig voneinander ausgewählt sind aus der Gruppe bestehend aus -H, -COOH, -(C₁-C₂₅)-Alkyl, -(C₁-C₂₅)-Alkyl-COOH, -O-(C₁-C₂₅)-Alkyl, -(C₁-C₂₅)-Alkyl-COO-(C₁-C₂₅)-Alkyl, -O-(CH₂-CH₂-O)_{b}-CH₃,-NH-CO-(C₁-C₂₅)-Alkyl, -NH₂, -NH-(C₁-C₂₅)-Alkyl, -N-((C₁-C₁₀)-Alkyl)₂ -CF₃, -Cl, -F, -CN, -NO ₂ und -CO-NH-(C₁-C₂₅)-Alkyl und
R5 gleich -(CH₂)_{d}-COO-R8 ist, wobei d eine ganze Zahl von 0 bis 10 ist, und R8 ausgewählt ist aus der Gruppe bestehend aus -H, -OH, -(C₁-C₈)-Alkyl, -(C₁-C₈)-Alkyl-OH, (C₁-C₈)-Acyl, -(CH₂-CH₂-O)_{b}-CH₃, wobei b eine ganze Zahl von 0 bis 20 ist, und einer Alkylkette, die mindestens eine Verknüpfung enthält, die in einer physiologischen Umgebung chemisch oder enzymatisch hydrolysiert werden kann.

8. Die flüssige polymere Zusammensetzung nach Anspruch 6, wobei das röntgendichte Molekül ausgewählt ist aus einer Verbindung der Formel III und einer Verbindung der Formel IV: 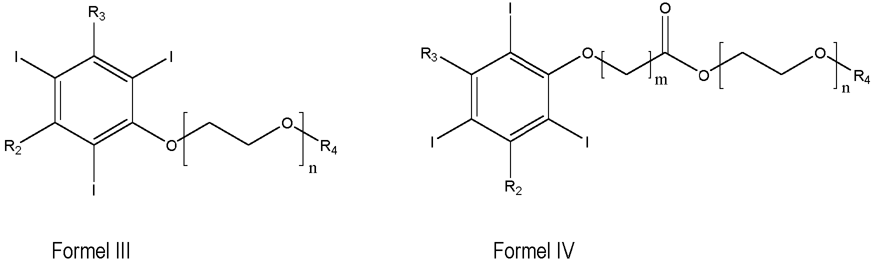 Wobei,
n und m unabhängig voneinander eine ganze Zahl von 1 bis 20 sind
R2 und R3 unabhängig voneinander ausgewählt sind aus der Gruppe bestehend aus -H, -COOH, -(C₁-C₂₅)-Alkyl, -(C₁-C₂₅)-Alkyl-COOH, -O-(C₁-C₂₅)-Alkyl, -(C₁-C₂₅)-Alkyl-COO-(C₁-C₂₅)-Alkyl, -O-(CH₂-CH₂-O)_{b}-CH₃,-NH-CO-(C₁-C₂₅)-Alkyl, -NH₂, -NH-(C₁-C₂₅)-Alkyl, -N-((C₁-C₁₀)-Alkyl)₂ -CF₃, -Cl, -F, -CN, -NO ₂ und -CO-NH-(C₁-C₂₅)-Alkyl und
R4 ausgewählt aus der Gruppe bestehend aus -H, -OH, -(C₁-C₈)-Alkyl, -(C₁-C₈)-Alkyl-OH, (C₁-C₈)-Acyl, -(CH₂-CH₂-O)b-CH₃ ist, wobei b eine ganze Zahl von 0 bis 20 ist, und einer Alkylkette, die mindestens eine Verknüpfung enthält, die in einer physiologischen Umgebung chemisch oder enzymatisch hydrolysiert werden kann.

9. Die flüssige polymere Zusammensetzung nach einem der Ansprüche 1 bis 8, wobei das röntgendichte Molekül ein endständiges Hydroxyl enthält.

10. Die flüssige polymere Zusammensetzung nach einem der Ansprüche 7 bis 9, wobei die mindestens eine Verknüpfung, die in einer physiologischen Umgebung chemisch oder enzymatisch hydrolysiert werden kann, aus der Gruppe ausgewählt ist, die aus einem Ester, einem Polyester, einem Amid und einem Carbonat besteht.

11. Die flüssige polymere Zusammensetzung nach einem der Ansprüche 1 bis 10, wobei das nicht-physiologische Lösungsmittel oder Lösungsmittelsystem ein organisches Lösungsmittel umfasst, das mit Wasser mischbar ist, insbesondere, wobei das nicht-physiologische Lösungsmittel oder Lösungsmittelsystem Dimethylsulfoxid, Dimethylformamid, einwertige Alkohole, mehrwertige Alkohole, ethoxylierte mehrwertige Alkohole, N-Methylpyrrolidon, Methylether von ethoxylierten mehrwertigen Alkoholen oder Kombinationen davon umfasst, wobei insbesondere das nicht-physiologische Lösungsmittel oder Lösungsmittelsystem Dimethylsulfoxid umfasst.

12. Die flüssige polymere Zusammensetzung nach einem der Ansprüche 1 bis 11, wobei die Viskosität der Zusammensetzung unter 50 cSt bei 40 °C liegt.

13. Ein injizierbares Mittel umfassend eine flüssige polymere Zusammensetzung wie in einem der Ansprüche 1 bis 12 definiert.

14. Eine flüssige polymere Zusammensetzung wie in einem der Ansprüche 1 bis 12 definiert oder ein injizierbares Mittel wie in Anspruch 13 definiert zur Verwendung beim Embolisieren eines Blutgefäßes.

## Revendications

1. Une composition polymère liquide comprenant :
(a) d'environ 2 à environ 20 pour cent en poids d'un polymère choisi dans le groupe constitué par un copolymère d'éthylène-alcool vinylique, l'acétate de cellulose, le poly(2-hydroxyéthylméthacrylate), les copolymères d'acétate de cellulose, le poly(2-hydroxyéthylméthacrylate), ou les deux, les copolymères de poly(oxyde d'éthylène)-co-poly(oxyde de propylène), les poly(oligoéthylène glycol)-(méth)acrylates, les copolymères de poly(méth)acrylate, les copolymères de poly(méth)acrylamide, et des combinaisons de ceux-ci ;
(b) d'environ 5 à environ 40 pour cent en poids d'une molécule radio-opaque ayant un poids moléculaire inférieur à 2000 g/mol ; et
(c) d'environ 40 à environ 93 pour cent en poids d'un solvant ou d'un système de solvant non physiologique ;
dans laquelle :
(i) la somme du pourcentage en poids de tous les composants de la composition est de 100,
(ii) le polymère et la molécule radio-opaque ne sont pas liés de manière covalente et sont tous deux dissous dans le solvant ou le système de solvant non physiologique,
(iii) le polymère est soluble dans la solution non physiologique et insoluble dans une solution physiologique à température ambiante, et
(iv) la composition polymérique liquide est capable de former, au contact avec des conditions physiologiques, un précipité radio-opaque qui conserve au moins 50 % de sa radio-opacité pendant au moins 30 minutes et perd au moins 50 % de sa radio-opacité en moins de 6 mois, en particulier, le précipité conserve au moins 50 % de sa radio-opacité pendant au moins 1 heure et perd au moins 50 % de sa radio-opacité en moins de 3 mois.

2. La composition polymère liquide selon la revendication 1, dans laquelle la composition polymère liquide forme un précipité avec une radiopacité homogène lors du contact avec des conditions physiologiques.

3. La composition polymère liquide selon l'une quelconque des revendications 1 à 2, dans laquelle le composé radio-opaque est une molécule aromatique qui, dans la composition, a un LogP de 2,0 à 9,0.

4. La composition polymère liquide selon l'une quelconque des revendications 1 à 3, dans laquelle le composé radio-opaque est une molécule aromatique non ionisable qui, dans la composition, a un LogP de 2,0 à 7,0, en particulier de 3,0 à 5,0, ou une molécule aromatique ionisable qui, dans la composition, a un LogP de 4,0 à 9,0, en particulier de 5,0 à 8,0, et comprend des groupes acides ou basiques.

5. La composition polymère liquide selon l'une quelconque des revendications 1 à 4, dans laquelle la molécule radio-opaque est une molécule aromatique contenant de l'iode ou du brome à une concentration supérieure à 300 mg équivalent d'iode ou de brome par gramme de molécule.

6. La composition polymère liquide selon l'une quelconque des revendications 1 à 5, dans laquelle la molécule radio-opaque est un dérivé pégylé de triiodophénol, d'acide iophénoxique, d'acide triiodobenzoïque, d'acide 3,5-diiodo-4-pyridone diatrizoïque, d'acide 5-amino-2,4,6-triiodoisophtalique ou de triiodoaniline.

7. La composition polymère liquide selon l'une quelconque des revendications 1 à 6, dans laquelle la molécule radio-opaque est un composé de formule I ou de formule Il 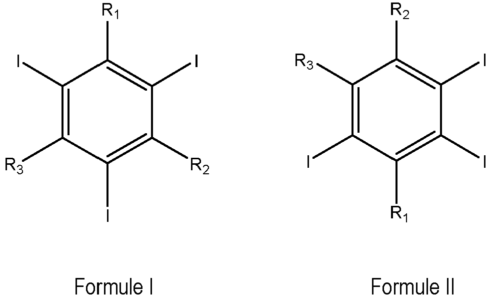 dans laquelle :
R2 et R3 sont choisis indépendamment dans le groupe constitué par -H, -COOH, -alkyle en (C₁-C₂₅), -alkyle en (C₁-C₂₅)-COOH, -O-alkyle en (C₁-C₂₅), alkyle en -(C₁-C₂₅)-COO-alkyle en (C₁-C₂₅), -O-(CH₂-CH₂-O)_{b}-CH₃, - NH-CO-alkyle en (C₁-C₂₅), -NH₂, -NH-alkyle en (C₁-C₂₅), -N-(alkyle en (C₁-C₁₀))₂ -CF₃, -CI, -F, -CN, -NO₂ et - CO-NH-alkyle en (C₁-C₂₅), et
R1 est choisi indépendamment dans le groupe constitué par
-X-alkyle en (C₁-C₂₀)-COO-R4
-X-(CH₂-CH₂-O)_{b}-R4,
-X-(CH₂-CH₂-O)_{b}-(CH₂)_{c}-COO-R4,
-X-(CH₂-CH₂-O)_{b}-COO-R4,
-X-alkyle en (C₁-C₁₀)-COO-(CH₂-CH₂-O)_{b}-R4,
-X-alkyle en (C₁-C₂₀)-NH₂,
-X-alkyle en (C₁-C₁₀)-NH-alkyle en (C₁-C₁₀)
-X-alkyle en (C₁-C₁₀)-N-(alkyle en (C₁-C₁₀))₂
-(CH₂)_{c}-O-(CH₂-CH₂-O)_{b}-R4,
où
X est choisi indépendamment dans le groupe constitué par O, COO et C(O)NH-
R4 est choisi dans le groupe constitué par -H, -OH, -alkyle en (C₁-C₈), -alkyle en (C₁-C₈)-OH, acyle en (C₁-C₈), -(CH₂-CH₂-O)_{b}-CH₃, et une chaîne alkyle contenant au moins une liaison capable d'être hydrolysée chimiquement ou enzymatiquement dans un environnement physiologique,
b est un nombre entier de 0 à 20, et
c est un nombre entier de 0 à 10.
ou, en variante, la molécule radio-opaque est un composé de formule V : 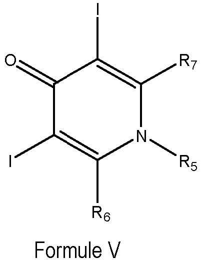
dans laquelle,
R6 et R7 sont choisis indépendamment dans le groupe constitué par -H, -COOH, -alkyle en (C₁-C₂₅), -alkyle en (C₁-C₂₅)-COOH, -O-alkyle en (C₁-C₂₅), -alkyle en (C₁-C₂₅)-COO-alkyle en(C₁-C₂₅), -O-(CH₂-CH₂-O)_{b}-CH₃,-NH-CO-alkyle en (C₁-C₂₅), -NH₂, -NH-alkyle en (C₁-C₂₅), -N-((alkyle en (C₁-C₁₀))₂ -CF₃, -CI, -F, -CN, -NO ₂ et - CO-NH-alkyle en (C₁-C₂₅), et
R5 est -(CH₂)_{d}-COO-R8, où d est un nombre entier de 0 à 10 et R8 est choisi dans le groupe constitué par -H, -OH, -alkyle en (C₁-C₈), alkyle en -(C₁-C₈)-OH, acyle en (C₁-C₈), -(CH₂-CH₂-O)_{b}-CH₃, où b est un nombre entier de 0 à 20, et une chaîne alkyle contenant au moins une liaison capable d'être hydrolysée chimiquement ou enzymatiquement dans un environnement physiologique.

8. La composition polymère liquide selon la revendication 6, dans laquelle la molécule radio-opaque est choisie parmi un composé de formule III et un composé de formule IV : 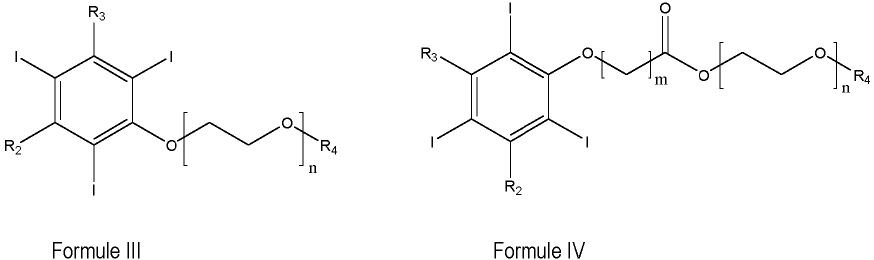 Dans lequel,
n et m sont indépendamment un nombre entier de 1 à 20
R2 et R3 sont choisis indépendamment dans le groupe constitué par -H, -COOH, -alkyle en (C₁-C₂₅), -alkyle en (C₁-C₂₅)-COOH, -O-alkyle en (C₁-C₂₅), -alkyle en (C₁-C₂₅)-COO-alkyle en (C₁-C₂₅), -O-(CH₂-CH₂-O)_{b}-CH₃,-NH-CO-alkyle en (C₁-C₂₅), -NH₂, -NH-alkyle en (C₁-C₂₅), -N-((alkyle en (C₁-C₁₀))₂ -CF₃, -CI, -F, -CN, -NO ₂ et - CO-NH-alkyle en (C₁-C₂₅), et
R4 est choisi dans le groupe constitué par -H, -OH, -alkyle en (C₁-C₈), -alkyle en (C₁-C₈)-OH, acyle en (C₁-C₈), -(CH₂-CH₂-O)_{b}-CH₃, où b est un nombre entier de 0 à 20, et une chaîne alkyle contenant au moins une liaison capable d'être hydrolysée chimiquement ou enzymatiquement dans un environnement physiologique.

9. La composition polymère liquide selon l'une quelconque des revendications 1 à 8, dans laquelle la molécule radio-opaque contient un hydroxyle terminal.

10. La composition polymère liquide selon l'une quelconque des revendications 7 à 9, dans laquelle la au moins une liaison capable d'être hydrolysée chimiquement ou enzymatiquement dans un environnement physiologique est choisie dans le groupe constitué par un ester, un polyester, un amide et un carbonate.

11. La composition polymère liquide selon l'une quelconque des revendications 1 à 10, dans laquelle le solvant ou système de solvant non physiologique comprend un solvant organique qui est miscible avec l'eau, en particulier, dans laquelle le solvant ou système de solvant non physiologique comprend du diméthylsulfoxyde, du diméthylformamide, des alcools monohydriques, des alcools polyhydriques, des alcools polyhydriques éthoxylés, de la N-méthylpyrrolidone, des éthers méthyliques d'alcools polyhydriques éthoxylés, ou des combinaisons de ceux-ci, en particulier, le solvant ou système de solvant non physiologique comprend du diméthylsulfoxyde.

12. La composition polymère liquide selon l'une quelconque des revendications 1 à 11, dans laquelle la viscosité de la composition est inférieure à 50 cSt à 40 °C.

13. Un produit injectable comprenant une composition polymère liquide telle que définie dans l'une quelconque des revendications 1 à 12.

14. Une composition polymère liquide telle que définie dans l'une quelconque des revendications 1 à 12 ou un produit injectable tel que défini dans la revendication 13 pour une utilisation dans l'embolisation d'un vaisseau sanguin.
